(19)
Europäisches
Patentamt
European
Patent Office
Office européen
des brevets

(11) **EP 2 274 081 B1**

(12) **EUROPÄISCHE PATENTSCHRIFT**

(45) Veröffentlichungstag und Bekanntmachung des Hinweises auf die Patenterteilung:
**18.10.2017 Patentblatt 2017/42**

(21) Anmeldenummer: **09733199.5**

(22) Anmeldetag: **10.02.2009**

(51) Int Cl.:
***B01D 69/02*** $^{(2006.01)}$ ***B01J 20/28*** $^{(2006.01)}$
***B01D 67/00*** $^{(2006.01)}$ ***B01D 71/10*** $^{(2006.01)}$
***B01J 20/26*** $^{(2006.01)}$ ***B01J 20/286*** $^{(2006.01)}$
***C07K 1/30*** $^{(2006.01)}$ ***B01J 20/285*** $^{(2006.01)}$

(86) Internationale Anmeldenummer:
**PCT/EP2009/000913**

(87) Internationale Veröffentlichungsnummer:
**WO 2009/127285 (22.10.2009 Gazette 2009/43)**

(54) **CELLULOSEHYDRAT-MEMBRAN, VERFAHREN ZU IHRER HERSTELLUNG UND VERWENDUNG DAVON**

CELLULOSE HYDRATE MEMBRANE, METHOD FOR THE PRODUCTION THEREOF, AND USE THEREOF

MEMBRANE EN HYDRATE DE CELLULOSE, SON PROCÉDÉ DE FABRICATION ET SON UTILISATION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **14.04.2008 DE 102008018719**
**04.11.2008 DE 102008055821**

(43) Veröffentlichungstag der Anmeldung:
**19.01.2011 Patentblatt 2011/03**

(73) Patentinhaber: **Sartorius Stedim Biotech GmbH**
**37079 Göttingen (DE)**

(72) Erfinder:
- **DEMMER, Wolfgang**
**37077 Göttingen (DE)**
- **FABER, René**
**37077 Göttingen (DE)**
- **HÖRL, Hans-Heinrich**
**37120 Bovenden (DE)**
- **NUßBAUMER, Dietmar**
**37079 Göttingen (DE)**

(74) Vertreter: **Müller-Boré & Partner**
**Patentanwälte PartG mbB**
**Friedenheimer Brücke 21**
**80639 München (DE)**

(56) Entgegenhaltungen:
**WO-A-03/015902** **WO-A-2007/017085**
**WO-A-2008/095709** **DE-A1- 4 323 913**
**US-A- 5 739 316** **US-A- 6 103 121**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die folgende Erfindung betrifft eine Cellulosehydrat-Membran, Verfahren zu ihrer Herstellung und die Verwendung davon als Adsorptionsmembran.

Der Erfindungsbeschreibung liegen folgende Begriffsbestimmungen und Sachverhalte zugrunde, wobei unter "Durchfluss" die hydraulische Permeabilität verstanden wird.

Als Adsorptionsmembranen werden flächige Adsorbentien mit von der einen Seite zur anderen Seite durchgehenden Poren bezeichnet. Adsorbentien sind poröse Feststoffe, die über als Liganden bezeichnete funktionelle Oberflächengruppen mit bestimmten Komponenten von Fluiden selektiv Bindungen eingehen können. Zielsubstanz(en) und/oder Kontaminant(en) werden erfindungsgemäß als Adsorbenden bezeichnet, wobei es sich auch um mehrere unterschiedliche Substanzen handeln kann. Adsorbenden können Einzelmoleküle, Assoziate oder Partikel sein, bei denen es sich vorzugsweise um Proteine oder andere Substanzen biologischen Ursprungs handelt.

Beispielhaft können als mit den bzw. dem Adsorbenden wechselwirkende Liganden Ionenaustauscher, Chelatbildner und Schwermetallchelate, thiophile, hydrophobe Liganden verschiedener Kettenlängen und Konfigurationen, Reversed Phase-Systeme, Farbstoffliganden, Affinitätsliganden, Aminosäuren, Coenzyme, Cofaktoren und deren Analoga, Substrate und deren Analoga, endokrine und exokrine Substanzen, wie Hormone und hormonähnlich wirkende Wirkstoffe, Effektoren und deren Analoga, Enzym-Substrate, Enzym-Inhibitoren und deren Analoga, Fettsäuren, Fettsäurederivate, konjugierte Fettsäuren und deren Analoga, Nukleinsäuren, wie DNA, RNA und deren Analoga und Derivate (einzel-, doppel-und/oder mehrsträngig), sowie Peptidnukleinsäuren und deren Derivate, Viren, Virenpartikel, Monomere und deren Analoga und Derivate, Oligo-bis Polymere und deren Analoga und Derivate, hochmolekulare Kohlenhydrate, die linear oder verzweigt, nicht-substituiert oder substituiert sein können, polymere Glycokonjugate, wie Heparin, Amylose, Cellulose, Chitin, Chitosan und deren Mono- und Oligomere und Derivate und Analoga davon, Lignin und dessen Derivate und Analoga, andere biologisch-chemische Liganden, wie Oligo- und Polypeptide, z.B. Proteine und ihre Oligomere, Multimere, Untereinheiten sowie Teile davon, insbesondere Lectine, Antikörper, Fusionsproteine, Haptene, Enzyme und Untereinheiten sowie Teile davon, Strukturproteine, Rezeptoren und Effektoren sowie Teile davon, des weiteren Xenobiotika, Pharmazeutika und pharmazeutische Wirkstoffe, Alkaloide, Antibiotika, Biomimetika usw. genannt werden.

Ein Adsorbens kann gleichzeitig auch zwei oder mehrere Arten der funktionellen Gruppen auf seiner inneren und äußeren Oberfläche tragen.

Die Bindung der Adsorbenden an das Adsorbens kann reversibel oder irreversibel sein, in jedem Fall ermöglicht sie ihre Abtrennung von den Fluiden, bei denen es sich im allgemeinen um wässrige Flüssigkeiten handelt und die im folgenden Medien genannt werden. Unter dem Begriff "Elution" werden die Desorption und die damit einhergehenden Spülschritte etc. zusammengefasst, und die zur Elution verwendete Flüssigkeit ist das "Eluens". Die Komponenten können eine oder mehrere Zielsubstanzen und/oder einen oder mehrere Kontaminanten darstellen. "Zielsubstanzen" sind Wertstoffe, die aus dem Medium in angereicherter oder reiner Form gewonnen werden sollen. Zielprodukte können beispielsweise rekombinante Proteine, wie z.B. monoklonale Antikörper sein. "Kontaminanten" sind Stoffe, deren Abwesenheit oder Entfernung aus dem Fluid aus technischen, regulatorischen oder sonstigen Gründen erforderlich oder wünschenswert ist. Kontaminanten können beispielsweise Viren, Proteine, Aminosäuren, Nukleinsäuren, Endotoxine, Proteinaggregate, Liganden oder deren Teile sein. Für die Entfernung von Kontaminanten, die als "negative Adsorption" bezeichnet wird, kann (darf) die Adsorption irreversibel verlaufen, wenn das Adsorbens nur einmal verwendet werden soll. Bei der Adsorption der Zielsubstanz(en) muß der Vorgang reversibel verlaufen. Es kann entweder eine bloße Anreicherung oder eine Auftrennung in mehrere Zielsubstanzen durchgeführt werden, wobei im letzteren Fall entweder die Adsorption, die Desorption oder beide selektiv erfolgen können.

Den Vorgang bezeichnet man als adsorptive Stofftrennung oder Chromatographie. Herkömmliche Adsorbentien für die Chromatographie sind partikulär geformt und werden in Form von Schüttungen in Säulen betrieben. Im Gegensatz dazu erfolgt die Anwendung von Adsorptionsmembranen im allgemeinen in Modulen, deren Bauformen den meist in der Membranfiltration üblichen entsprechen (z.B. Wickelmodul, Stapelmodul etc.). Die Anforderungen an die mechanische Festigkeit gleichen den an Filtrationsmembranen zu stellenden und sind damit wesentlich höher, als bei partikulären Adsorbentien, für die sich fragile Trägermaterialien, wie Gele von Dextran oder Agarose so universell etabliert haben, daß sich für sie der Begriff "Gele" als Synonym eingebürgert hat. Allen Membranen und Gelen ist dagegen eine Grundanforderung gemeinsam, und zwar die nach möglichst niedriger unspezifischer Adsorption.

Die Ausführung chromatographischer Trennung mit Hilfe von Adsorptionsmembranen wird auch Membranchromatographie genannt und sämtliche der in der Chromatographie bekannten synthetischen und natürlichen Liganden können in gleicher Weise auch für Adsorptionsmembranen eingesetzt werden. Der Bindung des Liganden an den Träger kann eine "Aktivierung" des Trägers vorausgehen, also die Einführung von reaktiven, funktionellen Gruppen, die zur spontanten Bindung des Liganden befähigt sind. Seltener weist der Ligand selbst eine reaktive Gruppe auf, wie z.B. die als Farbstoffliganden dienenden Reaktivfarbstoffe aus der Textilindustrie. Methoden zur Bindung von funktionellen Gruppen sind dem Fachmann an sich bekannt (z.B. aus Greg T. Hermanson, A. Krishna Mallia, Paul K. Smith, Immobilized Affinity Ligand Techniques, Academic Press, INC, 1992).

Die Menge an Adsorbend, bezogen auf die Menge des Adsorbens, die pro Beladung im Gleichgewicht mit dem Medium gebunden wird, d.h. die spezifische Bindungskapazität des Adsorbens, ist bei gegebener Ligandendichte proportional zu seiner spezifischen Oberfläche. Die spezifische Oberfläche von porösen Strukturen steigt mit abnehmender Porengröße, infolgedessen steigt auch ihre spezifische Bindungskapazität, solange die Ausschlußgrenze der Poren, also diejenige Molmasse, oberhalb derer ein Eintritt eines Moleküls möglich ist, die Molmasse des Adsorbenden nicht unterschreitet.

Adsorptionsmembranen bieten im Unterschied zu partikulären Adsorbentien die Möglichkeit, durch Anlegung einer hydraulischen Druckdifferenz zwischen den beiden Seiten ihrer Fläche eine Durchströmung mit dem Medium zu erzwingen, wodurch anstelle eines rein diffusen Transportes der Adsorbenden in Richtung eines Konzentrationsgradienten ins Innere des Adsorbens ein konvektiver Transport erreicht wird, der bei hohem Durchfluß sehr viel rascher erfolgen kann. Dadurch kann ein den partikulären Adsorbentien inhärenter Nachteil, der als "Diffusionslimitierung" bezeichnet wird, vermieden werden, der darin besteht, dass mit zunehmender Partikelgröße des Adsorbens und zunehmender Molmasse des Adsorbenden die erforderliche Zeit zur Einstellung des Adsorptionsgleichgewichts erheblich zunimmt, was sich in einer Verschlechterung der Kinetik auswirkt.

Die Ausnutzung des konvektiven Stofftransports mit Adsorptionsmembranen stößt jedoch insofern an Grenzen, als die Auswirkungen der Porengröße auf die Bindungskapazität und den Durchfluss gegensätzlich sind, wobei der Durchfluss mit zunehmender Porengröße ansteigt, die Bindungskapazität jedoch, wie erwähnt, abnimmt. Die Trennleistung von porösen Adsorptionsmembranen hängt sowohl von der Porenstruktur der Basismembran als auch der Art, Menge und Verteilung von funktionellen Gruppen in dieser Porenstruktur ab. Im Unterschied zu Filtrationsmembranen, für deren Leistungsfähigkeit vorwiegend die Struktur der durchgehenden Poren maßgebend ist, weist eine ideale Adsorptionsmembran also daneben eine möglichst große Anzahl solcher Poren auf, die die Ausschlussgrenze für den Adsorbenden nur wenig überschreiten und auch "Sackporen" sein können, also solche Poren, die nur eine einzige Verbindung mit einem durchströmten Bereich der Membran aufweisen. Solche Poren sind zwar nicht vollständig frei von der Diffusionslimitierung, weisen aber diesen Effekt nur in dem Maße auf, in dem der Stofftransport vom durchströmten Bereich durch freie Diffusion erfolgt. Diese unterschiedlichen Anforderungen an die Porenstruktur sind der Hauptgrund dafür, daß eine leistungsfähige Filtrationsmembran nicht allein durch Einführung entsprechender Liganden zu einer leistungsfähigen Adsorptionmembran wird.

Bei der Herstellung von Cellulosehydrat-Filtrationsmembranen aus Celluloseester-Membranen wird der Verseifungsprozess bevorzugt so durchgeführt, dass sich die poröse Struktur der Membran möglichst nicht ändert. Dies wird durch Maßnahmen erreicht, die einer Quellung der Cellulose entgegenwirken, z.B. durch Zugabe von Elektrolyten oder Alkoholen in das Verseifungsmedium.

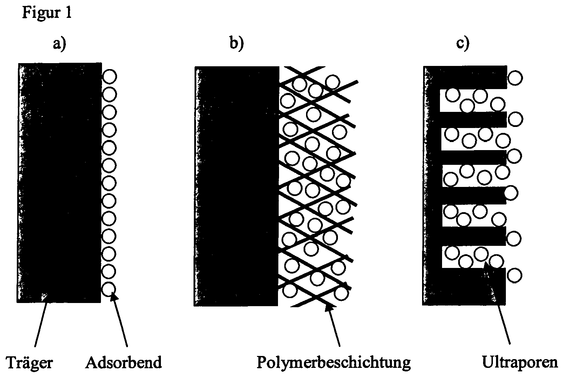

In WO 2007/017085 A2 wird ein Verfahren zur Herstellung von vernetzten Cellulosehydrat-Membranen beschrieben, das in der simultanen Verseifung und Vernetzung von Celluloseester-Membranen besteht und für Filtrations- und Adsorptionsmembranen gleichermaßen geeignet sein soll. Eines der Ziele der dort beschriebenen Erfindung ist die Verseifung und Vernetzung des Celluloseesters unter Bedingungen, die die Struktur und Permeabilität der Membran nicht beeinflussen. Da sich die Struktur der Membran im simultan ablaufenden Verseifungs- und Vernetzungsprozess nicht ändert, ist davon auszugehen, daß die Adsorption von Adsorbenden an der Oberfläche der Mikroporen des Trägers, die der Oberfläche der Ausgangs-Celluloseester-Membran entspricht, stattfindet (vgl. Figur 1a).

Darüber hinaus ist die Filtration, Aufreinigung oder Entfernung von Biomolekülen wie Proteinen, Aminosäuren, Nukleinsäuren, Viren oder Endotoxinen aus flüssigen Medien von großem Interesse für die biopharmazeutische Industrie. Die meisten Anwendungen der Kontaminantenentfernung werden z. Zt. mit konventionellen Chromatographiegelen betrieben. Die Chromatographiesäulen werden deutlich überdimensioniert, um ausreichende Flussraten zu erzielen. Die Säulen werden wiederverwendet, was einen erheblichen Reinigungs- und Validierungsaufwand bedeutet. Adsorptionsmembranen werden bereits in diesen Prozessen eingesetzt, wenn die Adsorbenden in der flüssigen Phase in sehr geringen Konzentration in Relation zur Kapazität der Matrix vorliegen, so dass bezogen auf die Flächeneinheit der Matrix bis zur Erschöpfung der Kapazität ein großes Volumen der flüssigen Phase verarbeitet werden kann. Typische Anwendungen sind im Bereich der negativen Adsorption, z. B. die Entfernung von Kontaminanten wie DNS, Viren, Wirtszellproteinen ("Host-Cell Proteins" (HCP), CHOP (Chinese Hamster Ovary Proteins), Endotoxinen aus antikörperhaltigen Lösungen mit positiv geladenen Membranen. Die Wirtszellproteine stellen ein breites Spektrum an unterschiedlichen Zellproteinen mit unterschiedlichen isoelektrischen Punkten (pl) und unterschiedlicher Größe und Affinität zum Adsorbens dar. Die Konzentration und Zusammensetzung der Kontaminanten hängen vom Expressionssystem und von den vorgeschalteten Aufreinigungsschritten ab. Typische Konzentrationen von Wirtszellproteinen in einem Protein-A-Pool liegen im Bereich von 500-5000 ppm (ng/mg Antikörper) und nach einem weiteren CEX-Schritt (Kationenaustauscher-Schritt) im Bereich von 50-500 ppm. Dies entspricht 0,5-5 g Wirtszellproteinen pro 10 kg Antikörper. Die Bindungskapazitäten von im Stand der Technik bekannten Anionenaustauschermembranen, z.B. Sartobind® Q der Fa. Sartorius Stedim Biotech GmbH, liegen im Bereich von 20-50 g BSA (Bovines Serumalbumin)/ l Membrane. Beim Einsatz eines 5-l-Membranadsorbers ist der Kapazitätsüberschuss 20-500-fach. Nach solcher Modellberechnung ist die Kapazität eines Membranadsorbers ausreichend, um alle Wirtszellproteine nach Protein-A- / CEX-Schritten vollständig zu entfernen. Durch das breite Spektrum an Wirtszellproteinen, von denen ein Teil je nach den Betriebsbedingungen entweder nicht geladen oder gleichsinnig wie das Adsorbens geladen ist, kommt es oft zum sofortigen Durchbruch von Wirtszellproteinen bereits bei niedrigen Ausnutzungsraten (ca. 1 %) des Adsorbens.

EP 0 586 268 B1 beschreibt ein Material zur Entfernung von Viren aus einer proteinhaltigen Lösung, umfassend ein Basismaterial, eine an das Basismaterial gebundene Oberflächenpfropfkette und eine über die Oberflächenpfropfkette auf der Oberfläche des Basismaterials mittelbar immobilisierte Polyaminverbindung. EP 0 586 268 B1 offenbart eine selektive Entfernung von pathogenen Substanzen, wie Leukozyten, Thrombozyten und Viren, aus Körperflüssigkeiten, wie Blut oder Plasma, wobei das offenbarte Material kein(e) Protein(e) aus den Körperflüssigkeiten adsorbieren soll. Polyaminverbindungen wie Spermidin, Spermin, Polyethylenimine unterschiedlichen Molekulargewichts zwischen Mw 300 - 70.000, Polyallylamin, Cationon UK, Panfix PX oder Poly-N-benzylvinylpyridinium-chlorid werden auf einer gepfropften Membran, z.B. aus Polypropylen oder PVDF (Polyvinylidenfluorid), immobilisiert. Eine Anwendung des offenbarten Materials ist die selektive Entfernung von Viren aus proteinhaltigen Lösungen. Bei Verwendung von Membranen, auf deren Oberfläche Spermidin oder Polyethylenimine immobilisiert sind, werden Entfernungsraten für den Phagen ΦX174, für ein HIV-Virus bzw. für das Herpesvirus I (H. F.-Stamm) aus Humanplasma zwischen 90 % und 99.8 % angegeben (Example 5, 6, 7 und 8). Bei Verzicht auf die Polyaminfunktionalisierung des Materials (Comparative Example 1) oder bei Einführung von quartären Ammoniumgruppen in die Pfropfkette, wobei gleichfalls auf die Polyaminfunktionalisierung verzichtet wird (Comparative Example 2), werden Membranen erhalten, die nur noch Entfernungsraten zwischen 50 % und 99 % für das Herpes-Virus und ΦX174 aus PBS-Puffer (Puffer basierend auf phosphatgepufferter Kochsalzlösung) aufweisen und höchstens 50 % aus Humanplasma.

Weiterhin sind keine quantitativen Angaben zur Proteinbindung durch die erfindungsgemäßen Materialien angegeben. Die offenbarte Selektivität für die Virusabreicherung mit LRV (negativer dekadischer Logarithmus des Rückhaltevermögens) von bis zu 3 Logarithmusstufen bezieht sich nur auf die Filtration von proteinhaltigen Lösungen wie Humanplasma. Es ist zu erwarten, dass sich die beschriebenen Eigenschaften und Vorteile der offenbarten Materialien nur auf Humanplasma beziehen, aber keine allgemeine Geltung für andere proteinhaltige Lösungen in Anspruch nehmen können.

Aus dem Stand der Technik ist bekannt, dass mit quaternären Ammonium-Gruppen funktionalisierte Membranen geeignet sind für die Virenentfernung aus Proteinlösungen, z. B. bei der Aufreinigung von monoklonalen Antikörpern. Mit Membranen wie z. B. Sartobind® Q der Fa. Sartorius Stedim Biotech GmbH werden LRV von mindestens 5 erreicht.

In WO 2008/008872 A2 sind Membranen beschrieben, die zur irreversiblen Bindung von Viren unter gleichzeitiger elektrostatischer Abstoßung von basischen Proteinen befähigt sind. Niedermolekulare multimodale Liganden, die auf der Membranoberfläche immobilisiert sind, gehen starke Interaktionen mit einem Modell-Phagen ΦX174 ein und zeigen LRV von bis zu 5,9 (Agmatin in Tabelle 2) bei Salzkonzentrationen von bis zu 150 mM NaCl. Die maximalen Bindungskapazitäten für das Modellprotein Rinderserum-Albumin liegen jedoch nur bei maximal 1000 mg/m$^2$, um den Faktor 5-20 unter den im Stand der Technik bekannten Membranadsorbern z. B. Sartobind® Q. Die Daten zeigen, dass die beanspruchten Membranen zwar hohe Virenabreicherungsraten zeigen, aber für eine breitere Anwendung, z. B. Kontaminantenentfernung aus Antikörperlösungen, keine ausreichende Bindungskapazitäten für Proteine aufweisen.

US 7,396,465 B2 offenbart positiv geladene mikroporöse Membranen, umfassend ein poröses Substrat, wie z. B. eine Polyethersulfonmembran oder eine Membran auf Cellulosebasis, und eine aus Polyaminen herstellbare, vernetzte Beschichtung, welche Ammoniumgruppen aufweist, wobei jede Ammoniumgruppe durch einen polaren Abstandshalter kovalent mit dem Polymerrückgrat der vernetzten Beschichtung verknüpft ist. Unter anderem werden Polyethylenimine oder Copolymere aus Diallylamin- und Acrylsäurederivaten als Edukte für die vernetzte Beschichtung verwendet.

In US 2007/0256970 A1 sind poröse Medien offenbart, die poröses Polyethylen mit mindestens einer polymeren Beschichtung umfassen, wobei die mindestens eine polymere Beschichtung vernetzt ist und aus einem Polyallylamin und aus mit Epichlorhydrin modifizierten Polyethyleniminen herstellbar ist.

Partikuläre Ionenaustauscher-Gele, die kovalent gebundene, polymere Amine an der Oberfläche eines porösen Basismaterials tragen, sind im Stand der Technik bekannt. In U.-J. Kim, S. Kuga, Journal of Chromatography A, 955 (2002), 191-196, sind Cellulose-Gele mit immobilisiertem Polyallylamin beschrieben, die durch partielle Oxidation von Cellulose mit Natriumperiodat und durch nachfolgende Schiffsche-Basenbildung mit Polyallylamin herstellbar sind.

In EP 1 224 975 B1 sind poröse Anionenaustauscherpartikel beschrieben, an deren Oberfläche ein Polyamin mit einem Zahlenmittel des Molekulargewichts von mindestens 50.000 gebunden ist.

EP 0 343 387 B1 beschreibt ein Assaysystem für Makromoleküle, bestehend aus mikroporösen Membranen mit einem kationischen Ladungsmodifizierungsmittel, das an die gesamte benetzte Oberfläche der Membran gebunden ist, wobei das kationische Ladungsmodifizierungsmittel das Reaktionsprodukt aus einem Polyamin mit Epichlorhydrin ist und tertiäre Amin- oder quaternäre Ammoniumgruppen sowie Epoxidgruppen längs der Polyaminkette enthält. Die Bindung des Ladungsmodifizierungsmittels an die Membran erfolgt über die Epoxidgruppen.

WO 03/015902 A2 betrifft eine Cellulosemembran, wobei die Membran aus einem Material gegossen worden ist, das ein Cellulosepolymer und ein Lösungsmittel umfasst, wobei die Membran eine erste poröse Fläche mit einem ersten durchschnittlichen Porendurchmesser, eine zweite poröse Fläche mit einem zweiten durchschnittlichen Porendurchmesser, und eine poröse Trägerstruktur dazwischen aufweist, wobei die Trägerstruktur ein netzförmiges Netzwerk aus Strömungskanälen umfasst, wobei der erste und der zweite durchschnittliche Porendurchmesser eine Asymmetrie von mindestens etwa 2:1 aufweisen, und wobei die porösen Flächen und die poröse Trägerstruktur ein Netzwerk von strukturellen Oberflächen aufweisen, die einen Filterstrom kontaktieren können.

US 6,103,121 betrifft ein Verfahren zur Herstellung eines Sorptionsmittels auf Membranbasis, das für eine Metallionenkomplexierung geeignet ist, umfassend das selektive Hydrolysieren einer mikroporösen Membran, die aus einem polyacetylierten Cellulosematerial aufgebaut ist, um eine Oberflächenschicht der Membran zu deacetylieren und freie Hydroxylgruppen freizulegen, das Oxidieren der freigelegten Hydroxylgruppen zu Aldehydgruppen und das Binden einer Polyaminosäure an die Cellulosemembran mittels der Aldehydgruppen.

DE 43 23 913 A1 beschreibt Trennmaterialien für die hydrophobe Chromatographie auf der Grundlage von hydroxylgruppenhaltigen Basisträgern, auf deren Oberflächen Polymere kovalent gebunden sind.

WO 2008/095709 A1, welche Stand der Technik gemäß Art. 54(3) EPÜ darstellt, beschreibt ein Verfahren zur Herstellung einer porösen, vernetzten, geladenen Cellulosepolymermembran, wobei die Cellulosepolymermembran in Gegenwart einer Base vernetzt wird und anschließend geladene Liganden in diese Membran eingeführt werden, und die so erhaltene Membran in Wasser eine Quellung von 40 bis 250 Vol.-% aufweist.

US 5,739,316 offenbart ein Verfahren zur Herstellung einer vernetzten Cellulosehydratmembran, umfassend das Inkontaktbringen einer Cellulosehydratmembran mit einer wässrigen alkalischen Lösung eines wasserlöslichen Diepoxids.

Konvektiv permeable Adsorbentien, die eine schnelle Kontaminantenabreicherung für ein breites Spektrum von Kontaminanten und Betriebsbedingungen, wie pH und Salzkonzentration, erlauben, sind von großem Interesse für biopharmazeutische Aufreinigungsprozesse.

Der Erfindung liegt daher die Aufgabe zugrunde, Membranen bereitzustellen, die aufgrund ihrer porösen Struktur und ihrer hohen mechanischen und chemischen Stabilität als Basismembranen für Adsorptionsmembranen mit hoher hydraulischer Permeabilität und Bindungskapazität geeignet sind sowie ein kostengünstiges und umweltfreundliches Verfahren zur Herstellung solcher flächigen Adsorbentien anzugeben. Darüber hinaus ist es insbesondere Aufgabe der vorliegenden Erfindung, Membranen bereitzustellen, die im Vergleich zu den im Stand der Technik bekannten Adsorbentien eine verbesserte Kontaminantenentfernung bei einem breiten Spektrum von Betriebsbedingungen erlauben.

Diese Aufgaben werden durch die in den Ansprüchen gekennzeichneten Gegenstände gelöst.

Die erfindungsgemäße Adsorptionsmembran umfaßt eine Cellulosehydrat-Matrix mit Poren, die sich von einer Hauptoberfläche zur anderen Hauptoberfläche der Membran erstrecken, wobei die Membran auf ihren inneren und äußeren Oberflächen funktionelle Gruppen (Liganden) zur adsorptiven Stofftrennung aufweist. Unter den Hauptoberflächen sollen die äußeren Flächen einer Membran verstanden werden.

In einer bevorzugten Ausführungsform der erfindungsgemäßen Membran sind die Mikroporen, die sich von der ersten Hauptoberfläche der Membran durch die Membran zur zweiten Hauptoberfläche erstrecken, unter Ausbildung von Kanälen kommunizierend miteinander verbunden und die Ultraporen erstrecken sich sackförmig von der inneren Oberfläche der Mikroporen in das die Struktur der Membran bildende Material hinein und/oder verbinden benachbarte Mikroporen miteinander.

Als Ausgangsmaterial für die erfindungsgemäße Adsorptionsmembran dient eine Celluloseester-Membran, die mit mindestens einer Lösung unter Bedingungen in Kontakt gebracht wird, die zum einen zu einer Quellung der Celluloseester-Matrix führen und zum anderen gleichzeitig, d.h. in situ, zur Hydrolyse (Verseifung) der Estergruppen zu Hydroxylgruppen, wobei eine Cellulosehydrat-Membran entsteht.

Die Quellung der Celluloseester-Matrix während der Hydrolyse (Verseifung) der Estergruppen wird durch den Quellungsgrad, d.h. das Verhältnis der Wasserpermeabilität der zuvor mit Wasser benetzten Celluloseester-Membran zur Wasserpermeabilität der endgültigen, d.h. verseiften, gegebenenfalls aktivierend vernetzten und mit Ligand(en) versehenen Cellulosehydrat-Membran, beschrieben.

Anschließend an die Verseifung wird die erhaltene Cellulosehydrat-Matrix durch Umsetzung der Hydroxylgruppen mit einem oder mehreren, mindestens bifunktionellen Reagens vernetzt und danach werden in die vernetzte Matrix bevorzugt funktionelle Gruppen (Liganden) zur Befähigung der adsorptiven Stofftrennung eingeführt.

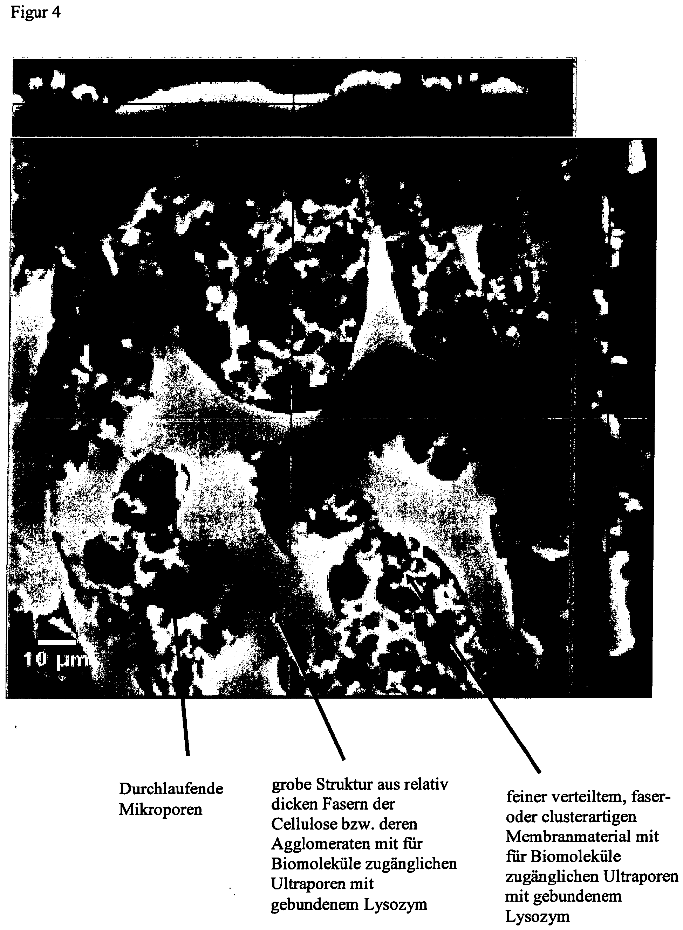

Überraschenderweise wurde festgestellt, daß die Bindungskapazität der Cellulosehydrat-Membran deutlich erhöht wird, wenn der Verseifungsschritt unter Bedingungen durchgeführt wird, unter denen die Cellulose quellen kann. Die Erhöhung der Bindungskapazität für Biomoleküle könnte auf die erhöhte Anzahl an für Biomoleküle zugänglichen, amorphen Bereichen der Cellulose zurückgeführt werden. Durch die Quellung des Celluloseträgers entstehen zwei Arten von Poren: a) Mikroporen mit einem Durchmesser von >100 nm, die in der Regel kleiner als die Originalporen der Celluloseester-Membran sind, und b) Ultraporen (amorphe Bereiche der Cellulose) mit einem Durchmesser von < 100 nm, die dergestalt sind, daß sie für Dextranblau (erhältlich als Blue Dextran molecular weight 2,000,000 von der Firma Sigma, St. Louis, Missouri, USA, Produktnummer D5751, CAS-Nummer: 87915-38-6) nicht zugänglich sind und die eine zusätzliche, für Liganden und Adsorbenden zugängliche Adsorptionsfläche anbieten (vgl. Figur 1c). Die Adsorptionswirksamkeit der erfindungsgemäßen Membran ist nicht auf die Phasengrenzfläche der verbundenen Mikroporen mit dem Medium beschränkt, sondern erstreckt sich zumindest auf einen Teil oder sogar das gesamte Volumen in den Ultraporen des Trägers (siehe Figur 4). Figur 4 zeigt eine konfokale Mikroskopieaufnahme einer mit Lysozym beladenen, erfindungsgemäßen Membran mit Ultraporen.

Die Quellung der Cellulose während der Verseifung kann durch eine geeignete Vorbehandlung des Celluloseesters oder durch die Parameter der Verseifung (Zusammensetzung des Verseifungsmediums, Art des Additivs, Konzentration des Additivs, Verseifungstemperatur) beeinflußt und gesteuert werden. So können die Permeabilität und Bindungskapazität der Membran eingestellt werden. Die in dem erfindungsgemäßen Verfahren hergestellten, adsorptiven Cellulosehydrat-Membranen zeigen gegenüber den durch auf dem Fachgebiet bekannte Herstellungsverfahren hergestellten Cellulosehydrat-Membranen deutlich höhere Bindungskapazitäten bei vergleichbaren Permeabilitäten.

Wie im Weiteren beschrieben wird, kann das Verfahren zur Herstellung der erfindungsgemäßen Membran in drei Schritten durchgeführt werden, wobei die Einstellung des gewünschten Quellungsgrads, des Durchflusses und der Bindungskapazität sowohl durch die Parameter der Vorbehandlung (Art des Additivs, Konzentration des Additivs, Vorbehandlungstemperatur) als auch die Parameter der Verseifung (Zusammensetzung des Verseifungsmediums, Art des Additivs, Konzentration des Additivs, Verseifungstemperatur) gesteuert werden kann. Die erfindungsgemäße Membran kann auch ohne Vorbehandlung der Celluloseester-Matrix hergestellt werden. Hohe Quellungsgrade der Cellulosehydrat-Matrix können gemäß dem erfindungsgemäßen Verfahren durch hohe Konzentration an Alkalimetallhydroxid im Verseifungsmedium, hohe Konzentration an wasserstoffbrückenbrechenden Verbindungen oder niedrige Temperatur des Verseifungsmediums erreicht werden.

Durch die Art des Vernetzungsmittels, die Konzentration des Vernetzungsmittels, die Konzentration des Vernetzungskatalysators, die Vernetzungsdauer, gegebenenfalls die Art und die Konzentration eines inerten organischen Lösungsmittels und/oder die Vernetzungstemperatur kann der Vernetzungsgrad, die Porengröße und die Anzahl an restlichen, aktiven Gruppen, z.B. Epoxid-Gruppen, gesteuert werden. Dadurch kann die für die Bindung der funktionellen Gruppen oft notwendige Aktivierung bereits in dem Vernetzungsschritt stattfinden.

In einem weiteren Schritt können funktionelle Gruppen, z.B. an die Hydroxylgruppen, der vernetzten Membran gebunden werden. Methoden zur Bindung von funktionellen Gruppen sind dem Fachmann an sich bekannt (z.B. aus Greg T. Hermanson, A. Krishna Mallia, Paul K. Smith, Immobilized Affinity Ligand Techniques, Academic Press, INC, 1992).

Bevorzugt werden funktionelle Gruppen über Epoxid-Gruppen oder Aldehyd-Gruppen an die Cellulosemembran gebunden. Die Einführung der Epoxid-Gruppen kann bereits in dem Vernetzungsschritt oder nachträglich stattfinden.

Die Kombinationen der Einflußgrößen (a) der Herstellungsbedingungen der als Ausgangsmaterial verwendeten Celluloseester-Membran, (b) der Bedingungen der gegebenenfalls durchgeführten Vorbehandlung, (c) der Verseifungsbedingungen und (d) der Vernetzungsbedingungen der Celluloseester-Membran, ermöglichen es auch, aus einer Ausgangsmembran mehrere unterschiedliche Endprodukte herzustellen, was produktionstechnisch eine erhebliche Vereinfachung zur Folge hat.

**Ausgangsmembran**

Die in dem erfindungsgemäßen Verfahren als Ausgangsmembran verwendete Celluloseester-Membran mit einer Porengröße von 0,1 bis 20 μm, vorzugsweise 0,5 bis 15 μm und mehr bevorzugt von 1 bis 10 μm wird durch ein übliches, auf dem Fachgebiet bekanntes Herstellungsverfahren hergestellt. Zur Bestimmung der Porengröße wird ein "Capillary Flow Porometry Test" durchgeführt. Weitere Details sind der Bedienungsanleitung (Capillary Flow Porometer 6.0, CAPWIN Software System, Fa. Porous Materials Inc.) zu entnehmen. Celluloseester-Membranen können aus Cellulosemonoacetat, Cellulosediacetat, Cellulosetriacetat, Cellulosepropionat, Cellulosebutyrat und Celluloseacetobutyrat oder anderen geeigneten Celluloseestern oder Cellulosenitrat, Methylcellulose oder Ethylcellulose, sowie Gemischen davon, aufgebaut sein, wobei Celluloseacetate, insbesondere Cellulosediacetat, bevorzugt sind bzw. ist. Dem Fachmann ist bekannt, daß die Celluloseester-Membran zum Teil auch Hydroxylgruppen neben den Estergruppen enthalten kann.

**Vorbehandlung**

Vor dem Verseifen kann die Celluloseester-Membran in einem geeigneten Medium vorbehandelt werden. Die Temperatur in dem Vorbehandlungsschritt liegt vorzugsweise in einem Bereich von 20 bis 100°C, wobei eine Temperatur in einem Bereich von etwa 60°C bis etwa 80°C besonders bevorzugt ist. Als Vorbehandlungsmedium kann ein Gas, wie beispielsweise Luft, ein organisches Lösungsmittel, wie beispielsweise ein Alkohol, oder ein wässriges Medium verwendet werden, wobei ein wässriges Medium bevorzugt ist. Das Vorbehandlungsmedium enthält vorzugsweise ein oder mehrere Additiv(e), das bzw. die eine gegenüber einem Celluloseester lösende oder weichmachende Wirkung aufweist bzw. aufweisen. Geeignete Additive sind vor allem Säuren, insbesondere Carbonsäuren, wie Essigsäure, und wasserlösliche Weichmacher für Celluloseester, wie Diacetin, Triacetin und Sulfolan. Es ist jedoch vor allem aus wirtschaftlichen Gründen besonders bevorzugt, Essigsäure als Additiv für das Vorbehandlungsmedium zu verwenden, obwohl auch Diacetin und Triacetin ausgezeichnete Ergebnisse liefern, jedoch teurer sind. Die Konzentration des Additivs in dem Vorbehandlungsmedium unterliegt keinen besonderen Beschränkungen.

Die Dauer der Vorbehandlung hat keinen wesentlichen Einfluß auf den Vorbehandlungseffekt, sofern eine Mindesteinwirkdauer angewendet wird, die eine Temperaturangleichung der Celluloseester-Membran in dem Vorbehandlungsmedium und eine Konzentrationsangleichung des gegebenenfalls verwendeten Additivs in der Membran gewährleistet. Die obere Grenze der Einwirkungsdauer des Vorbehandlungsmediums ist durch jene Zeit bestimmt, ab der eine chemische Umsetzung der Celluloseestermembran mit dem Vorbehandlungsmedium, beispielsweise durch Hydrolyse, eintreten könnte. In anderen Worten ausgedrückt, wird die Einwirkungsdauer des Vorbehandlungsmediums derart eingestellt, daß keine (frühzeitige) Hydrolyse oder Verseifung der vorbehandelten Celluloseester-Membran eintritt. Üblicherweise beträgt die Einwirkungsdauer des Vorbehandlungsmediums auf die Celluloseester-Ausgangsmembran zwischen 0,1 Sekunden und 1 Stunde, wobei eine Einwirkungsdauer von 10 Sekunden bis 10 Minuten bevorzugt ist. Das Ausmaß des Vorbehandlungseffekts ist abhängig von der höchsten Temperatur in Verbindung mit der höchsten Konzentration des Additivs, die auf die Celluloseester-Membran einwirken. Wenn also die Abkühlung oder Ausspülung des Additivs über einen längeren Zeitraum erfolgt, ist das ohne Einfluß auf den bereits erreichten Vorbehandlungseffekt. Die Beendigung der Vorbehandlung kann daher durch Ausspülen des Vorbehandlungsadditivs aus der Membran und/oder Absenkung der Temperatur des Vorbehandlungsmediums erfolgen.

**Verseifung**

Die gegebenenfalls vorbehandelte Celluloseester-Membran wird mit einem geeigneten Verseifungsmedium verseift, wodurch sich die Cellulosehydrat-Membran unter Quellung der Cellulose-Matrix ausbildet. Je nach der Art des Vorbehandlungsmediums kann die Celluloseester-Membran trocken oder naß im Verseifungsschritt eingesetzt werden.

Durch die Quellung der Cellulose wird die Zugänglichkeit der Hydroxylgruppen für die Anbindung der funktionellen Gruppen und anschließend für die Adsorbenden verbessert. Das Verseifen der Cellulose-Ester-Membran wird in einem wässrigen Medium mit einem pH-Wert von >7, d.h. ein basisches Medium, durchgeführt, wobei das Verseifungsmedium Natrium- oder Lithiumhydroxid enthält. Es können auch Gemische aus Natrium- oder Lithiumhydroxid und anderen alkalischen Verbindungen wie Alkalimetallcarbonat, wie Natriumcarbonat, Kaliumcarbonat, Cäsiumcarbonat, Natriumhydrogencarbonat, und/oder Natriumtriphosphat, Kaliumtriphosphat, Natriumsilikat und Kaliumsilicat eingesetzt werden.

Die Konzentration des Natrium- oder Lithiumhydroxids im Verseifungsmedium beträgt 0,4 bis 50 Gew.-%, bezogen auf das Verseifungsmedium, und besonders bevorzugt 0,4 bis 10 Gew.-%. Gemäß einer besonders bevorzugten Ausführungsform der vorliegenden Erfindung wird ein Verseifungsmedium aus Wasser und Natriumhydroxid verwendet, wobei die Konzentration des Natriumhydroxids in dem Verseifungsmedium in einem Bereich von 0,4 bis 20 Gew.-%, besonders bevorzugt in einem Bereich von 0,4 bis 4 Gew.-% liegt.

Das Verseifungsmedium kann ein oder mehrere Additiv(e) enthalten, das bzw. die eine gegenüber einem Celluloseester quellungsbeeinflussende Wirkung aufweist bzw. aufweisen. Geeignete Additive sind vor allem Salze, wie Natriumchlorid, Natriumsulfat und Natriumacetat, Wasserstoffbrücken brechende Verbindungen, wie Harnstoff oder organische Lösungsmittel, wie Ethylamin. Das organische Lösungsmittel wird vorzugsweise aus der Gruppe von Alkohlen, Ketonen oder Ethern ausgewählt. Besonders bevorzugt ist Ethanol, Methanol, Ethylenglycol, Propylenglycol, Glycerin, Aceton, Dioxan oder Diglyme. Das Additiv in dem Verseifungsmedium sollte die Quellung beeinflussen, aber nicht vollständig unterdrücken.

Die Temperatur des verwendeten Mediums in dem Verseifungsschritt kann von etwa 10°C bis zum Siedepunkt des Verseifungsmediums betragen, wobei eine Temperatur in einem Bereich von 15°C bis etwa 25°C bevorzugt ist.

Die Verseifungsdauer richtet sich nach der Zusammensetzung des Verseifungsmediums und der Verseifungstemperatur. Üblicherweise beträgt die Verseifungsdauer 0,1 bis 60 Minuten, wobei eine Verseifungsdauer von 5 bis 45 Minuten bevorzugt ist. Besonders bevorzugt ist eine Verseifungsdauer von 20 bis 40 Minuten.

Die erhaltene Cellulosehydrat-Membran kann jede geeignete Dicke aufweisen. Vorzugsweise liegt die Schichtdicke im Bereich zwischen 50 und 500 μm, noch bevorzugter im Bereich zwischen 100 und 300 μm. Die erhaltene Cellulosehydrat-Membran kann eben oder auch zylindrisch ausgebildet sein. Zylindrische Membranen werden als Membranhohlfasern, Membrankapillaren oder Membranschläuche bezeichnet.

**Vernetzung**

Anschließend wird die nach der gegebenenfalls durchgeführten Vorbehandlung und der unter Quellung stattgefundenen Verseifung erhaltene Cellulosehydrat-Membran mit einem Vernetzungsmittel zur Erhöhung der chemischen Widerstandsfähigkeit der Membran und/oder zur Einführung von funktionellen Gruppen vernetzt.

Das Vernetzungsmittel weist mindestens zwei funktionelle Gruppen im Molekül auf, die mit den Hydroxylgruppen von Cellulose reaktiv sind und somit eine Vernetzung von Cellulose ermöglichen. Die verwendbaren Vernetzungsmittel unterliegen grundsätzlich keinen besonderen Beschränkungen und ein Fachmann ist in der Lage, sie aus einer Reihe von für die Vernetzung von Cellulose verwendbaren

Vernetzungsmitteln auszuwählen. Es ist jedoch bevorzugt, in dem Vernetzungsschritt eine Diepoxidverbindung oder auch andere, mit Hydroxylgruppen von Cellulose reaktive Verbindungen mit mindestens zwei reaktiven, funktionellen Gruppen, wie Diisocyanat, Epichlorhydrin, Epibromhydrin, Dimethylharnstoff, Dimethylethylenharnstoff, Dimethylchlorsilan, Bis(2-hydroxyethylsulfon), Divinylsulfon, Alkylen-Dihalogen, Hydroxyalkylen-Dihalogen und Glycidylether zu verwenden.

Aus der Gruppe der Glycidylether sind 1,4-Butandioldiglycidylether, Ethylenglycoldiglycidylether, Glycerindiglycidylether und Polyethylenglycoldiglycidylether bevorzugt.

Besonders bevorzugt ist die Verwendung von 1,4-Butandioldiglycidylether oder Epichlorhydrin als Vernetzungsmittel.

Optional kann ein Gemisch von unterschiedlichen Vernetzungsmitteln verwendet werden.

Die Vernetzung kann in einem wässrigen Medium, in einem organischen Lösungsmittel oder auch in einem Gemisch aus Wasser und einem organischen Lösungsmittel stattfinden. Vorzugsweise wird die Vernetzung in einem wässrigen Medium durchgeführt.

Ferner ist es bevorzugt, einen Vernetzungskatalysator, wie Natriumhydroxid, zur Beschleunigung der Vernetzung von Cellulose mit dem Vernetzungsmittel zu verwenden.

Die Temperatur des verwendeten Mediums in dem Vernetzungsschritt kann von etwa 4°C bis zum Siedepunkt des Vernetzungsmediums betragen, wobei eine Temperatur in einem Bereich von 5°C bis etwa 70°C bevorzugt ist. Besonders bevorzugt ist eine Temperatur von 20°C bis 40°C.

Üblicherweise beträgt die Vernetzungsdauer 10 Minuten bis 100 Stunden, wobei eine Verseifungsdauer von 30 Minuten bis 48 Stunden bevorzugt ist. Besonders bevorzugt ist eine Verseifungsdauer von 2 bis 24 Stunden.

Wie vorstehend beschrieben, kann das Verfahren zur Herstellung der erfindungsgemäßen Membran in drei Schritten durchgeführt werden, wobei die Einstellung des gewünschten Quellungsgrads der Matrix sowohl durch die Parameter der Vorbehandlung (Art des Additivs, Konzentration des Additivs, Vorbehandlungstemperatur) als auch die Parameter der Verseifung (Zusammensetzung des Verseifungsmediums, Art des Additivs, Konzentration des Additivs, Verseifungstemperatur) gesteuert werden kann. Die erfindungsgemäße Membran kann auch ohne Vorbehandlung hergestellt werden.

**Aktivierung und Bindung von funktionellen Gruppen**

In einem weiteren Schritt können funktionelle Gruppen an die Hydroxylgruppen der vernetzten Cellulosehydrat-Membran gebunden werden. Methoden zur Bindung von funktionellen Gruppen sind dem Fachmann bekannt (z.B. aus Greg T. Hermanson, A. Krishna Mallia, Paul K. Smith, Immobilized Affinity Ligand Techniques, Academic Press, INC, 1992). Pfropfverfahren von funktionellen Monomeren oder Polymeren und Polymerbeschichtungsverfahren sind dem Fachmann bekannt und können zur Einführung von funktionellen Gruppen verwendet werden.

Bevorzugt werden funktionelle Gruppen über Epoxid-Gruppen oder Aldehyd-Gruppen an die Cellulosemembran gebunden. Die Epoxid-Aktivierung kann bereits in dem Vernetzungsschritt oder nachträglich stattfinden.

Es ist auch möglich, funktionelle Gruppen während der Vernetzung einzuführen, z.B. durch Zugabe von einem Amin und/oder von einer monofunktionellen Epoxidverbindung, wie Phenylglycidylether oder Butylglycidylether zu einer Diepoxidverbindung.

Gemäß einer bevorzugten Ausführungsform der vorliegenden Erfindung sind die funktionellen Gruppen Liganden, die mit in Fluiden enthaltenen Adsorbenden Wechselwirkungen eingehen.

Vorzugsweise können die funktionellen Gruppen Bestandteil eines oligomeren oder polymeren Abstandshalters sein, welcher die funktionellen Gruppen mit der Cellulosemembran verknüpft.

Besonders bevorzugt handelt es sich bei den funktionellen Gruppen um Liganden, welche vorzugsweise anionische oder kationische Gruppen umfassen. Bei den anionischen Gruppen kann es sich z.B. um Sulfon-, Phosphor- oder Carbonsäuren handeln und bei den kationischen Gruppen um primäre, sekundäre, tertiäre und/oder quaternäre Amine.

Dabei ist es insbesondere bevorzugt, dass die kationischen Gruppen primäre, sekundäre und/oder tertiäre Amine sind, wobei die primären, sekundären und/oder tertiären Amine monomere oder polymere Amine sein können. Die primären, sekundären und/oder tertiären Amine sind vorzugsweise polymere Verbindungen mit linearen und/oder verzweigten und/oder cyclischen Strukturen. Im Rahmen der vorliegenden Erfindung werden unter polymeren Aminen Polyamine mit mindestens einer primären, sekundären und/oder tertiären Amingruppe in einer Polymerkette verstanden.

Polymere, die eine lineare, verzweigte oder cyclische Struktur aufweisen und die primäre, sekundäre und/oder tertiäre Amingruppen enthalten, eignen sich gut für eine kovalente Immobilisierung auf aktivierten Oberflächen. Solche Polymere bieten eine ausreichende Anzahl an kationischen Gruppen, die zur Adsorption von negativ geladenen Substanzen befähigt sind. Die direkte kovalente Anbindung von polymeren Aminen an porösen Trägern führt zu stabilen positiv geladenen Oberflächen. Polyamine sind multifunktionelle, mit kationischen Ladungen versehbare Polymere mit einer verzweigten, sphärischen Struktur. Wegen ihrer hohen Ladungsdichte adsorbieren diese Polymere stark an negativ geladenen Oberflächen, wie Cellulose, Polyester, Polyolefine, Polyamide und Metalle. Sie werden deshalb u. a. zur Vermittlung einer verbesserten Haftung zwischen unterschiedlichen Materialien eingesetzt.

Durch die Verwendung von polymeren Aminen als Liganden ist es möglich, eine Membran bereitzustellen, die im Vergleich zu den im Stand der Technik bekannten Absorbentien eine verbesserte Kontaminantenentfernung bei einem breiten Spektrum von Betriebsbedingungen aufweist. Dabei können die Kontaminanten jedwede Stoffe sein, deren Abwesenheit oder Entfernung aus einem Fluid aus technischen, regulatorischen oder sonstigen Gründen erforderlich oder wünschenswert ist. Vorzugsweise sind die Kontaminanten Viren, Proteine, Aminosäuren, Nukleinsäuren, Endotoxine, Proteinaggregate, Liganden oder deren Teile. Insbesondere ist die erfindungsgemäße Cellulosehydrat-Membran in Verbindung mit einer Polyamin-Funktionalisierung zur Kontaminantenentfernung geeignet, wenn hohe Bindungskapazitäten für Proteine erforderlich sind.

Dabei können alle geeigneten polymeren Amine verwendet werden. Bevorzugt sind jedoch polymere Amine, die vorwiegend primäre Amingruppen enthalten. Besonders bevorzugt sind polymere Amine, die vorwiegend primäre Amingruppen enthalten und ein Molekulargewicht von mehr als 500 g/mol aufweisen.

In einer besonders bevorzugten Ausführungsform der vorliegenden Erfindung sind die polymeren Verbindungen aus der Gruppe der Polyalkylenimine mit einer Molmasse zwischen 800 und 1.000.000 g/mol ausgewählt. Vorzugsweise ist das Polyalkylenimin ein Polyethylenimin. Polyethylenimine (PEI) fällen Nukleinsäuren und Proteine aus wässrigen Medien. Auf porösen Trägern immobilisiertes PEI filtriert beispielsweise effektiv Endotoxine und Pathogene aus Blutplasma.

In einer anderen besonders bevorzugten Ausführungsform sind die polymeren Verbindungen aus der Gruppe der Polyallylamine mit einer Molmasse zwischen 3.000 und 150.000 g/mol ausgewählt.

In einer weiteren bevorzugten Ausführungsform sind die polymeren Verbindungen aus der Gruppe der Polyvinylamine mit einer Molmasse zwischen 5.000 und 500.000 g/mol ausgewählt. Polyvinylamin weist die höchste kationische Ladungsdichte auf, die bisher bekannt ist. Bei höheren Hydrolysegraden nimmt die Ladungsdichte mit steigendem pH-Wert ab. Im Gegensatz zu anderen Polymeren besitzt Polyvinylamin jedoch auch bei hohen pH-Werten (pH = 9-10) eine signifikante Ladungsdichte von konstant ~6 meq/g. Bei Hydrolysegraden unter 50 % wird die Ladungsdichte nicht vom pH-Wert beeinflusst.

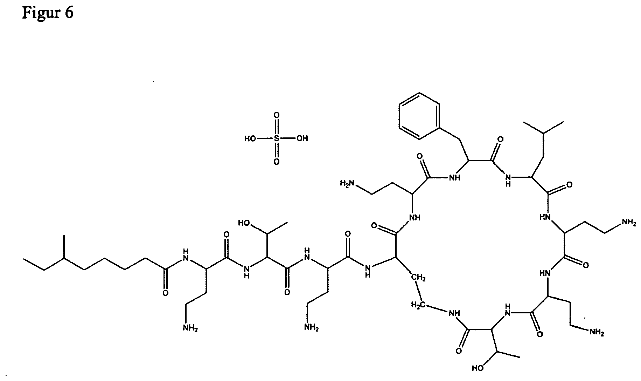

In einer weiteren bevorzugten Ausführungsform ist die polymere Verbindung Polymyxin B (vgl. Figur 6). Bei Polymyxin B handelt es sich um ein Cyclopeptid mit Fettsäurerest, welches bei physiologischem pH-Wert kationische Amingruppen aufweist und hydrophile und hydrophobe Eigenschaften hat.

Die polymeren Amine können entweder direkt an der Oberfläche des Trägers oder über einen Abstandshalter an den Träger gebunden werden oder über eine epoxygruppenhaltige Polymerschicht auf der Oberfläche des Trägers gebunden werden, wie z.B. durch polymeranaloge Umsetzung von Glycidylmethacrylatgepfropften (GylMA-gepfropften) Trägern mit den Polyaminen.

Unter "polymeranalogen Umsetzungen" sollen hierbei Reaktionen an Makromolekülen, bei denen die chemische Zusammensetzung und damit auch die Eigenschaften eines Polymers unter Erhalt des Polymerisationsgrades verändert werden, verstanden werden.

Methoden zur Immobilisierung von funktionellen Gruppen über Amino-Gruppen sind dem Fachmann bekannt. (Greg T. Hermanson, A. Krishna Mallia, Paul K. Smith, Immobilized Affinity Ligand Techniques, Academic Press, INC, 1992). Bevorzugt werden z.B. aldehyd- oder epoxyaktivierte Träger, die mit Aminen umgesetzt werden können, eingesetzt. Aktive Gruppen können bereits, wie vorstehend beschrieben, bei der Herstellung des porösen Trägers eingeführt werden.

In einer bevorzugten Ausführungsform der vorliegenden Erfindung kann eine Di- oder Triepoxidverbindung dem polymeren Amin vor oder während der Reaktion mit der Membran zugesetzt werden. Die Epoxidverbindung führt zur Vernetzung des polymeren Amins und zur kovalenten Bindung des Polyamins an die Trägeroberfläche. Additive wie Salze, Polymere oder organische Lösungsmittel können dem Polyamin und/oder dem Träger vor oder während der Reaktion des Polyamins mit der Trägeroberfläche zugegeben werden.

Die polymeren Amine werden vorzugsweise in wässrigen Lösungen mit den aktivierten Trägern zur Reaktion gebracht. Die Konzentration kann dabei beliebig variiert werden.

Es wurde jedoch festgestellt, dass eine Umsetzung von z.B. Glycidylmethacrylat-(GylMA)-gepfropften Polymerschichten mit einem polymeren Amin zur Vernetzung der Polymerschicht führt. Eine mehrseitige Reaktion ("multiple-point-attachment") des polymeren Amins in der GylMA-Polymerschicht kann entweder mit benachbarten Glycidyl-Gruppen jeweils einer Polymerkette intramolekular oder mit Glycidyl-Gruppen zweier Polymerketten intermolekular stattfinden. Die zweite Möglichkeit führt zur Vernetzung der Polymerschicht auf der Membran. Die Vernetzung verursacht, dass die Polyaminschicht für Biomoleküle nicht mehr zugänglich ist, was sich in niedrigen Bindungskapazitäten für Proteine zeigt (s. Vergleichsbeispiel 2). Diese Methode eignet sich daher nicht für die Immobilisierung von höhermolekularen polymeren Aminen.

In einer weiteren bevorzugten Ausführungsform der vorliegenden Erfindung ist mindestens eine primäre, sekundäre oder tertiäre Amingruppe des polymeren Amins mit einer Komponente, ausgewählt aus der Gruppe, umfassend Phenylglycidylether, 1,2-Epoxyethylbenzol, Dodecylglycidylether, Tetradecylglycidylether, Benzylchlorid, (3-Glycidyloxypropyl)trimethoxysilan, Bis[(4-glycidyloxy)phenyl]methan, Bisphenol-A-diglycidylether, 1,4-Butandiol-diglycidylether, 6-Hexandioldiglycidylether, N,N-(Diglycidyl-4-glycidyloxy)anilin, 4,4'-Methylen-bis(N,N-diglycidyl)anilin und/oder Tris(4-hydroxyphenyl)methan-triglycidylether umgesetzt. Auf diese Weise ist es möglich, die Affinität des Adsorbens für die Zieladsorbenden zu steuern. Dabei können die freien primären, sekundären oder tertiären Gruppen des polymeren Amins während oder nach der Reaktion mit dem porösen Träger mit den weiteren funktionellen Gruppen umgesetzt werden.

Bei allen Ausführungsformen, bei welchen mindestens ein polymeres Amin als Ligand an die erfindungsgemäßen Membranen gebunden ist, wird dieses direkt auf der Oberfläche des Trägers, d. h. ohne intermediäre Pfropfkette, immobilisiert.

Die Struktur der erfindungsgemäßen Membran erlaubt eine direkte Oberflächenanbindung von polymeren Aminen (ohne zusätzliche, intermediäre Pfropfketten) zur Bildung einer für Biomoleküle zugänglichen Polyaminschicht auf der Membranoberfläche.

Ein Vorteil der erfindungsgemäßen Membran besteht darin, dass durch die große Auswahl an monomeren oder polymeren Aminen und an hydrophoben funktionellen Gruppen der für die Umsetzung mit den polymeren Aminen einsetzbaren, zuvor genannten Verbindungen optimale Adsorbentien für die gewünschte Anwendung hergestellt werden können.

In einer weiteren bevorzugten Ausführungsform der vorliegenden Erfindung sind die Liganden Affinitätsliganden, z.B. p-Aminobenzamidin, biomimetische Liganden und/oder Proteine.

In einer weiteren bevorzugten Ausführungsform der vorliegenden Erfindung sind die Liganden ausgewählt aus den Metallchelaten.

In einer weiteren bevorzugten Ausführungsform der vorliegenden Erfindung sind die Liganden hydrophob und sind ausgewählt aus der Gruppe der $C_1$-$C_{20}$-Alkylgruppen, $C_6$-$C_{25}$-Arylgruppen, $C_7$-$C_{25}$-Arylalkylgruppen und deren Derivaten und/oder -$[(CH_2)_m$-O-$]_n$-R, wobei m 2 oder 3 ist, n eine ganze Zahl von größer als 1 ist und wobei R -H oder -$C_1$-$C_5$-Alkyl bedeuten.

In einer weiteren bevorzugten Ausführungsform der vorliegenden Erfindung sind die Liganden ausgewählt aus der Gruppe der reaktiven Epoxid-, Aldehyd-, Azlacton-, N-Hydroxysuccinimid- und/oder Carbodiimid-Gruppen.

Schließlich kann der mindestens eine Ligand in einer weiteren bevorzugten Ausführungsform der vorliegenden Erfindung ein Katalysator sein.

Der mindestens eine Ligand kann bevorzugt über einen oligomeren oder polymeren Abstandshalter mittelbar an die Cellulosemembran gebunden sein oder kann besonders bevorzugt unmittelbar an die Cellulosemembran gebunden sein.

Vorzugsweise sind mindestens zwei strukturell unterschiedliche Liganden an die erfindungsgemäße Membran gebunden.

Die erfindungsgemäßen Membranen können nach der Einführung von funktionellen Gruppen optional getrocknet werden. Membranen können direkt aus Wasser oder organischen Lösungsmitteln, bevorzugt Alkohol, getrocknet werden, oder nach einem stufenweise durchgeführten Austausch des Wassers durch ein organisches Lösungsmittel. Bevorzugt werden die Membranen aus einem Medium getrocknet, das eine porenstabilisierende Verbindung enthält. Besonders bevorzugt werden die erfindungsgemäßen Membranen aus wässriger Glycerinlösung getrocknet. Die Konzentration des Glycerins liegt vorzugsweise im Bereich von 5 bis 40 Gewichtsprozent, bezogen auf die wässrige Lösung.

**Erläuterung der Beispiele**

Vernetzte Cellulosehydrat-Membranen mit niedrigem Quellungsgrad werden beispielsweise aus mit ethanolischer Kalilauge verseiften Celluloseester-Membranen hergestellt. Eine Celluloseacetat-Membran ergibt auf diese Weise eine Cellulosehydrat-Membran mit geringfügig niedrigerem Durchfluss (siehe Beispiel 1), die aber nach Einführung von Liganden praktisch kein Adsorptionsvermögen (Bindungskapazität) aufweist (siehe Tabelle 2).

Es wurde nun gefunden, daß bei Verseifung mit wässriger Natronlauge zwar der Durchfluß sinkt (siehe Beispiel 2), nach Belegung mit diversen Liganden aber deutlich erhöhte Bindungskapazitäten auftreten, wobei steigende NatronlaugeKonzentrationen sich in Richtung einer stärkeren Durchflußminderung und höheren Bindungskapazitäten auswirken (siehe Tabelle 2). Gegenüber den durchgehenden Mikroporen, die sich bei der Verseifung mit ethanolischer Kalilauge vorwiegend bilden, scheint bei der Verseifung mit wässriger Natronlauge die Entstehung einer Vielzahl kleiner Ultraporen bevorzugt zu sein. Eine höhere Verseifungstemperatur sowie ein zusätzlicher Gehalt an Elektrolyten einschließlich bei der Verseifung bereits gebildeten Natriumacetats wirken sich dabei in der gleichen Richtung aus, wie eine niedrigere Natronlauge-Konzentration.

In WO 2007/017085 A2 wird ein Verfahren zur Herstellung von vernetzten Cellulosehydrat-Membranen beschrieben, das in der simultanen Verseifung und Vernetzung von Celluloseester-Membranen besteht und für Filtrations- und Adsorptionsmembranen gleichermaßen geeignet sein soll. Eines der Ziele der dort beschriebenen Erfindung ist die Verseifung und Vernetzung des Celluloseesters unter Bedingungen, die die Struktur und Permeabilität der Membran nicht beeinflussen. Durch gleichzeitige Verseifung und Vernetzung unter Bedindungen, die die Quellung und Strukturänderung unterdrücken ($Na_2SO_4$, niedrige Natronlauge-Konzentration) wird keine signifikante Bindungskapazität festgestellt (siehe Vergleichsbeispiel 1). Erst wenn die Laugenkonzentration erhöht wird, kommt es zur Erhöhung der Bindungskapazität. Durch die Quellung der Cellulose kommt es hier aber auch zur Änderung der Porenstruktur, was im Widerspruch zum in dem Stand der Technik beschriebenen, simultanen Verseifungs- und Vernetzungsprozess steht. Die Bindungskapazität beträgt hier aber nur ca. 5 % der Bindungskapazität im Vergleich zu der separat unter quellenden Bedingungen durchgeführten Verseifung und Vernetzung (siehe Beispiel 2 und Vergleichsbeispiel 1).

Weiterhin wurde gefunden, daß sich die unterschiedliche Vorbehandlung der Celluloseacetat-Membran unterschiedlich auf die Eigenschaften der erfindungsgemäßen adsorptiven Membran auswirkt. Der Durchfluss sinkt und die Bindungskapazität steigt, wenn die Celluloseacetat-Membran vor der Verseifung auf 80°C an der Luft erhitzt wurde (siehe Beispiel 3). Wenn die Celluloseacetatmembran vor der Verseifung in 20%iger Essigsäure auf 80°C erhitzt wird (siehe Beispiel 4), steigt der Durchfluss im Vergleich zu der nicht vorbehandelten Membran aus dem Beispiel 2 und die Bindungskapazität ändert sich abhängig von der Größe des Proteins. Für Lysozym (14,3 kDa) steigt die Bindungskapazität, für Rinderserumalbumin (RSA; 60 kDa) und Gamma-Globulin (150 kDa) sinkt die Bindungskapazität. Die Vorbehandlung kann beispielsweise bei speziellen Trennaufgaben dann vorteilhaft sein, wenn die Spezifität des Liganden für die Stofftrennung alleine nicht ausreicht und die Molmassen der zu trennenden Komponenten so stark unterschiedlich sind, daß sich aus der Überlagerung der rein adsorptiven Stofftrennung mit einem Größenausschlußeffekt insgesamt eine Verbesserung der Trennleistung ergibt, und die Beeinflussung der Porengröße der Ultraporen durch die Wahl der Base und deren Konzentration einer Unterstützung bedarf. Eine vollständige Trennung auf der Basis allein dieses Effekts ist allerdings nicht möglich, weil der Größenausschluß nur für die Adsorption an der inneren Oberfläche der Ultraporen wirksam wird, nicht aber an der äußeren Oberfläche der Mikroporen.

Diese Befunde lassen bei der Verseifung und Vernetzung von Celluloseacetaten auf komplexe Quellungs- und Entquellungsvorgänge schließen, deren Auswirkungen im Hinblick auf die Struktur des Endprodukts schwer zusammenfassend darzustellen sind, weil es sowohl Vorgänge gibt, bei denen eine Durchflussminderung mit einer Erhöhung der Bindungskapazität gekoppelt ist, als auch solche, bei denen das nicht der Fall ist. Die erstgenannten werden im folgenden als "produktiv" bezeichnet, die sonstigen als "unproduktiv". Die Vorbehandlung der Celluloseacetat-Membran wirkt sich unterschiedlich auf die Änderung der Porenstruktur, die Bildung von Mikroporen sowie von Ultraporen aus. So ist es möglich, durch geeignete Wahl der Vorbehandlung den Durchfluss, die Bindungskapazität aber auch den Größenausschluss der Ultraporen der erfindungsgemäßen adsorptiven Membran zu beeinflussen. Das Hauptziel des erfindungsgemäßen Verfahrens ist die Beschränkung auf produktive Durchflussminderungen, wobei nicht nur das Quellverhalten des Ausgangsmaterials, der Celluloseacetat-Membran, und des Endprodukts, der vernetzten Cellulosehydrat-Membran, in Betracht zu ziehen sind, sondern auch das gesamte Spektrum der Zwischenprodukte im teilverseiften und teilvernetzten Zustand. So ist beispielsweise bekannt, daß Celluloseacetate abnehmenden Acetylgehalts in einem engen Bereich sogar einen Zustand der Wasserlöslichkeit durchlaufen.

Erfindungsgemäß wird eine Celluloseester-Membran sequentiell in einem quellenden Medium verseift, mit einem mindestens bifunktionellen Agens vernetzt und vorzugsweise mit einem adsorptionswirksamen Liganden versehen. Das Quellvermögen der Alkalimetallhydroxide nimmt in Richtung kleinerer Kationenradien und höherer Konzentrationen zu (siehe Beispiel 5).

Die Vernetzung der Cellulose erfolgt erfindungsgemäß vorzugsweise mit 1,4-Butandioldiglycidylether. In einer Ausgestaltung der Erfindung erfolgt die Vernetzung der Cellulose mit 1,4-Butandioldiglycidylether in der Weise, dass infolge einer zum Teil einseitigen Reaktion eine ausreichende Zahl nicht umgesetzter Epoxidgruppen erhalten bleibt ("aktivierende Vernetzung", siehe Beispiel 2), die zur Bindung oder zur Ankopplung bzw. zum Aufbau von Liganden dienen können. Die nicht umgesetzten Epoxidgruppen sind relativ hydrolysestabil und wurden noch nach bis zu 24 Stunden Feuchtlagerung bei Raumtemperatur für Folgereaktionen eingesetzt. In einer anderen Ausführungsform der Erfindung zur Bindung "aktiver" Liganden wird die Vernetzung unter verschärften Bedingungen (längere Vernetzungsdauer und/oder höhere Alkalikonzentration und/oder höhere Temperatur) durchgeführt, so dass unter gesteigerter Reaktion mit der Cellulose und/oder gesteigerter Hydrolyse der überzähligen Gruppen ein Verbleib von Epoxidgruppen weitgehend unterbleibt ("nicht aktivierende Vernetzung", siehe Beispiel 6). Restliche Epoxidgruppen können auch durch nachträgliche Behandlung mit z.B. 5%iger Schwefelsäure bei erhöhter Temperatur hydrolisiert werden.

Der Durchfluß der erfindungsgemäßen Membran nach Beispiel 2 für 20 mM tris/HCl Puffer mit einem pH-Wert von 7,3 ist um 8% größer als der für reines Wasser. Entsprechende Werte von Adsorptionsmembranen, die nach dem Stand der Technik durch Beschichtung hergestellt wurden, liegen zwischen 20% im Fall eines vernetzten Hilfspolymeren und 200% bei einem unvernetzten. Die entstandene Porenstruktur gibt sich durch die geringe Abhängigkeit des Durchflusses von der Ionenstärke des Mediums als ein Hybrid aus Aerogel und Xerogel ähnlich wie bei einem vernetzten Agarosegel zu erkennen.

Die erfindungsgemäßen Adsorptionsmembranen lassen sich von nach dem Stand der Technik durch Polymerbeschichtung oder Pfropfung hergestellten Adsorptionsmembranen durch Rasterelektronenmikroskopie schwer unterscheiden, weil deren Auflösungsvermögen durch die den Hauptunterschied ausmachenden kleinporigen Strukturen (d.h. Ultraporen) überfordert wäre. Dagegen liefert die Charakterisierung von adsorptiven Membranen mittels der sogenannten konfokalen Laserfluoreszenzmikroskopie (CLSM) unter geeigneten Bedingungen simultan sowohl Information zur Porenstruktur als auch zur Verteilung von an funktionellen Gruppen gebundenem Protein in der Membran. Dafür müssen Membranmaterial und Protein mit zwei verschiedenen Fluoreszenzfarbstoffen markiert werden. Alle mikroskopischen Messungen wurden ungefähr im selben Abstand (ca. 20 μm) von der jeweiligen äußeren Oberfläche durchgeführt. In allen Fällen führten drei unabhängige Messungen an verschiedenen x, y-Positionen zu sehr ähnlichen, für den jeweiligen Membrantyp charakteristischen Ergebnissen.

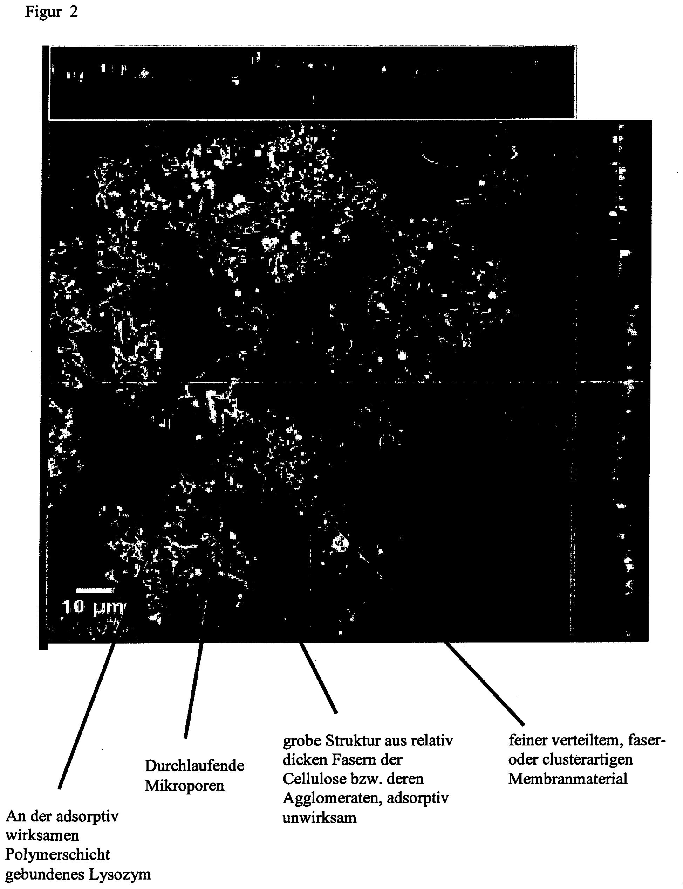

Charakteristisch für alle Membranproben ist eine sehr grobe Struktur (dunkle Bereiche in den Figuren 2 - 4) aus relativ dicken Fasern bzw. deren Agglomeraten mit feiner verteiltem Faser- oder clusterartigen Membranmaterial mit dazwischen sich befindlichen, vollständig oder teilweise ungefärbten Bereichen, die den durchlaufenden Mikroporen mit Dimensionen bis zu ca. 20 μm zugeordnet werden können. Die Proteinverteilung war für alle Membranproben eindeutig zu identifizieren. Allerdings wurden sehr große Unterschiede in Bezug auf Proteinmenge (Fluoreszenzintensität, helle Bereiche) und Proteinverteilung in der Porenstruktur (dunkle Bereiche) gefunden. Die Gesamt-Fluoreszenzintensitäten waren deutlich unterschiedlich, für die erfindungsgemäße Membran nach Beispiel 2 mußte sogar eine geringere Verstärkung als für die anderen Membranen gewählt werden:

Membran aus Beispiel 2 > Sartobind® S Membran >> Membran aus Beispiel 1

Diese Ergebnisse korrelieren gut mit den Angaben zur Bindungskapazität:

Membran nach Beispiel 1: 0,01 $mg/cm^2$
Membran Sartobind® S: 0,90 $mg/cm^2$
Membran nach Beispiel 2: 2,06 $mg/cm^2$

Mit der Untersuchungsmethode konnten eindeutige und große Unterschiede in Bezug auf die Proteinbindung zwischen der etablierten Sartobind® S Membran (Figur 2) sowie den aus dem Beispiel 1 (Figur 3) und dem Beispiel 2 (Figur 4) mit Sulfonsäureliganden funktionalisierten Membranen identifiziert werden. Bei den Membranen aus den Beispielen 1 und 2 korrelieren die Fluoreszenzintensitäten für das Protein (helle Bereiche) mit den nominalen Proteinbindungskapazitäten, d.h. die Membran aus dem Beispiel 1 weist eine nur sehr geringe Bindungskapazität auf, wobei die erfindungsgemäße Membran aus dem Beispiel 2 eine deutlich höhere Bindungskapazität aufweist.

Ausgehend von derselben Porenstruktur des Basisträgers bindet das Protein in den Sartobind® S Membranen vor allem im Volumen der Mikroporen, wobei wesentlich für die Proteinbindung eine dreidimensionale funktionelle Schicht ist. Dabei zeigen diese Membranen an den Rändern der Poren scharfe Abgrenzungen zwischen dem Material der Membran (dunkle Bereiche) und der Proteinschicht (helle Bereiche). Wegen der begrenzten Reichweite dieser funktionellen Schicht verbleiben kleine Anteile am Porenvolumen, in denen kein Protein gebunden ist. Bei der Membran aus dem Beispiel 1 findet die Bindung direkt am Membranmaterial statt, was durch kleine helle Punkte in der Figur 2 zu erkennen ist. Im Gegensatz dazu werden bei der erfindungsgemäßen Membran aus dem Beispiel 2 offensichtlich sehr große Mengen an Protein in den Ultraporen der groben Faserstruktur sowie in dem feiner verteilten, faser- oder clusterartigen Membranmaterial gebunden. Zwischen den Verteilungen von Cellulose und Protein wird eine sehr gute Korrelation gefunden, visuell auch daran erkennbar, daß in der Überlagerung nur die Mischfarbe der verwendeten Farbstoffe zu erkennen ist, weil in der Tiefe der Ultraporen sowohl Porenoberfläche als auch Protein sichtbar sind. Der weitaus größte Anteil des Volumens der Mikroporen enthält kein Protein.

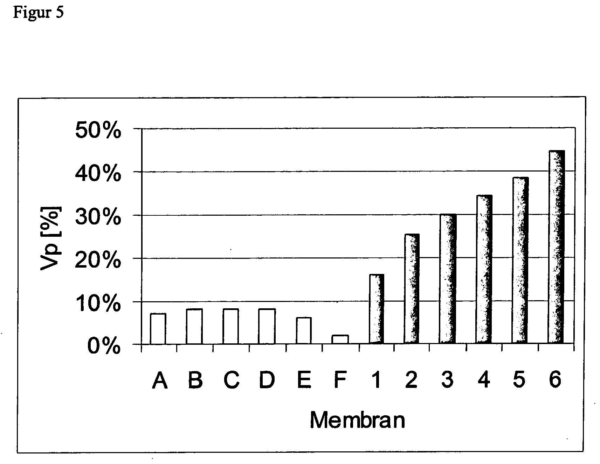

Um die Ultraporen der erfindungsgemäßen Membranen quantitativ zu erfassen, wurde ein Versuch durchgeführt, in dem die Porenzugänglichkeit für Dextranblau bestimmt wurde. Die Versuchsdurchführung erfolgte in der in Beispiel 14 beschriebenen Weise. Das Ergebnis der Auswertung ist in Tabelle 1 und in Figur 5 gezeigt.

In der Figur 5 ist ein deutlicher Unterschied zwischen den für Dextranblau unzugänglichen Ultraporen für die im Stand der Technik bekannten Membranen A-F und erfindungsgemäßen Membranen aus dem Beispiel 14 zu erkennen. Im Falle der erfindungsgemäßen Membranen, die unter quellenden Bedingungen verseift wurden, liegt mehr als 15% des gesamten Porenvolumens im Bereich von Ultraporen (d.h. Poren mit einem Durchmesser <100 nm, die für Dextranblau nicht zugänglich sind), wohingegen es bei den Vergleichsmembranen A-F weniger als 8% sind.

Demgemäß weisen erfindungsgemäße Membranen ein Volumen an Ultraporen, die zwar für Wasser zugänglich sind, jedoch nicht für Dextranblau mit einem Molekulargewicht Mw von 2.000.000, von mehr als 15%, vorzugsweise mehr als 18%, mehr bevorzugt von mehr als 20%, noch mehr bevorzugt von mehr als 25% und am meisten bevorzugt von mehr als 30% des gesamten Porenvolumens auf.

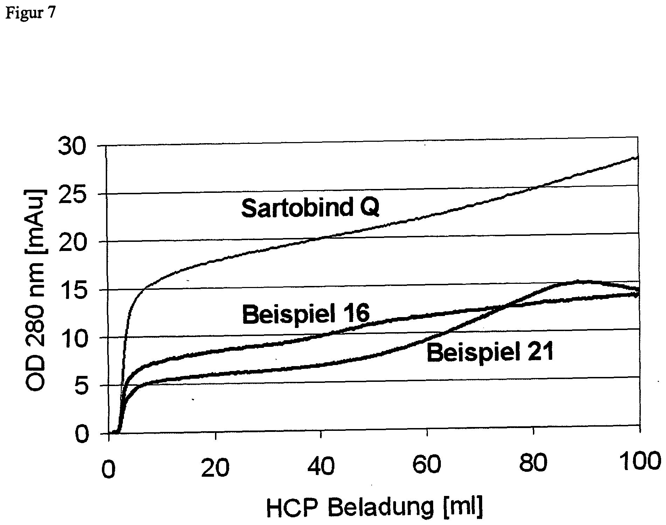

Darüber hinaus wurde gefunden, dass bei Einführung einer Polyamin-Funktionalisierung in die erfindungsgemäßen Membranen die Membranen im Vergleich zur Sartobind® Q-Membran aus dem Stand der Technik vergleichbare oder höhere Bindungskapazitäten für das Modellprotein Rinderserumalbumin (RSA), deutlich höhere Ladungsdichten und deutlich höhere dynamische Bindungskapazitäten für DNS als Modellkontaminante zeigen (vgl. Beispiele 15 bis 23 sowie Vergleichsbeispiele 3 und 4). Figur 7 zeigt Durchbruchskurven für ein Modell-HCP-Gemisch. Die Durchbruchskurven zeigen, dass die erfindungsgemäßen Membranen aus den Beispielen 16 und 21 deutlich höhere Affinitäten zu den Wirtszellproteinen aufweisen, da die Fraktion der gebundenen Proteine, die der Fläche oberhalb der Durchbruchskurve entspricht, größer ist als für die Sartobind® Q-Membran. Da es sich bei dem HCP-Gemisch um ein Gemisch von Proteinen unterschiedlicher Größe und isoelektrischer Punkte (d.h. Ladung) handelt (vgl. "Auswertung der Membranen (für die Beispiele 15 bis 23 und die Vergleichsbeispiele 3 und 4)", Abschnitt 5)), bedeutet die Verschiebung des Durchbruchs nach unten (Erhöhung der Fläche oberhalb der Durchbruchskurve) gegenüber der Sartobind® Q-Membran, dass mehrere unterschiedliche Proteine in größerer Menge an den erfindungsgemäßen Membranen binden. Dies liegt in Übereinstimmung mit dem Ziel der Erfindung.

Der Bacteriophage ΦX174 mit einem Durchmesser von ca. 30 nm und einem isoelektrischen Punkt pl von 6,4-6,7 wird als Modellvirus zur Untersuchung der Effizienz der Virenabreicherung verwendet. Seine Abreicherung mit den erfindungsgemäßen Membranen bei unterschiedlichen Salzkonzentrationen in einem Puffer im Vergleich zu der im Stand der Technik bekannten Sartobind® Q Membran wird untersucht ("6) Bindung von Modellviren"). Die Ergebnisse in der Tabelle 4 zeigen, dass die erfindungsgemäßen Membranen den Phagen bei hohen Salzkonzentrationen effektiv abreichern, wobei die Abreicherung mit der Sartobind® Q Membran nur bei niedrigen Salzkonzentrationen möglich ist. Dies ist in Übereinstimmung mit der Aufgabe der vorliegenden Erfindung, Membranen bereitzustellen, die im Vergleich zu den im Stand der Technik bekannten Adsorbentien eine verbesserte Kontaminantenentfernung bei einem breiten Spektrum von Betriebsbedingungen erlauben.

**Figuren**

Figur 1a): Schematische Darstellung der Proteinbindung an Mikroporen einer wie im Beispiel 1 hergestellten und im Stand der Technik bekannten adsorptiven Membran.

Figur 1b): Schematische Darstellung der Proteinbindung an einer adsorptiv wirksamen Polymerbeschichtung von wie in dem Stand der Technik beschriebenen adsorptiven Membranen, die aus einer oder mehreren Trägerstruktur(en) und einer oder mehreren adsorptiv wirksamen Polymerbeschichtung(en) bestehen.

Figur 1c): Schematische Darstellung der Proteinbindung in den Ultraporen einer erfindungsgemäßen adsorptiven Membran.

Figur 2: CLSM-Bild der Porenmorphologie und Proteinverteilung an der Oberseite der Sartobind® S Membran nach Markierung der Cellulose mit Fluoreszenzfarbstoff und Beladung mit Fluoreszenz-markiertem Lysozym.

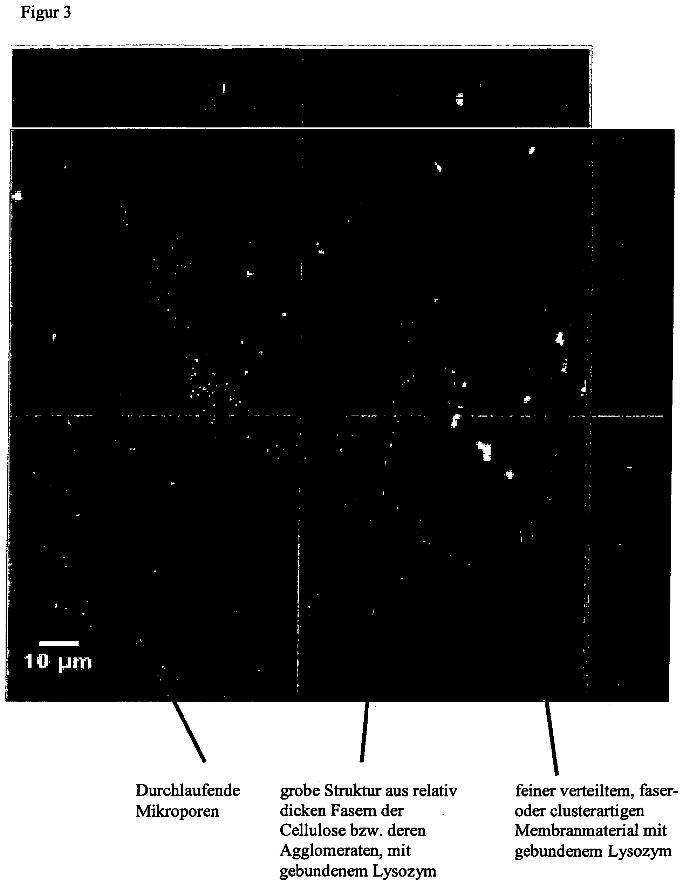

Figur 3: CLSM-Bild der Porenmorphologie und Proteinverteilung an der Oberseite der mit Sulfonsäureliganden umgesetzten Membran nach Beispiel 1 nach Markierung der Cellulose mit Fluoreszenzfarbstoff und Beladung mit Fluoreszenz-markiertem Lysozym.

Figur 4: CLSM-Bild der Porenmorphologie und Proteinverteilung an der Oberseite der mit Sulfonsäureliganden umgesetzten Membran nach Beispiel 2 nach Markierung der Cellulose mit Fluoreszenzfarbstoff und Beladung mit Fluoreszenz-markiertem Lysozym.

Figur 5: Vergleich des prozentualen Anteils von für Dextranblau mit einem Molekulargewicht Mw von 2.000.000 unzugänglichen Poren in den Membranen:

A: Membrane aus Beispiel 1
B-F: Cellulose-Membranen nach dem Stand der Technik 0,2-0,45 μm der Fa. Sartorius Stedim Biotech GmbH
1-6: Erfindungsgemäße Membranen aus dem Beispiel 14

Figur 6: Struktur von Polymyxin B

Figur 7: Durchbruchskurven für ein Modell-HCP-Gemisch für die Membran

Sartobind® Q und für die erfindungsgemäßen Membranen der Beispiele 16 und 21.

## Beispiele

Sämtliche Erwähnungen in den Beispielen einer CA-Membran beziehen sich auf einen mit Polyester-Vlies verstärkten Typ einer Celluloseacetat-Membran mit einem Porendurchmesser von ca. 3 μm (gemessen mit einem Coulter-Porometer Typ Capillary Flow Porometer 6.0, CAPWIN Software System, Fa. Porous Materials Inc.), welche einen Wasserdurchfluss von 730 ml/(min x bar x $cm^2$) aufweist. Die Dicke der modifizierten Membranproben betrug durchschnittlich 250 μm. Alle Durchflussangaben sind in ml/(min x bar x $cm^2$) und alle Bindungskapazitätsangaben sind in mg/$cm^2$. Sofern nichts anderes angegeben ist, beziehen sich Prozentangaben auf das Gewicht. Für die Beispiele 15 bis 23 und die Vergleichsbeispiele 3 und 4 wiesen die Membranen einen Wasserdurchfluss von 600 bis 700 ml/(min x bar x $cm^2$) auf.

### Beispiel 1

Aktivierend vernetzte Cellulosehydrat-Membran mit einem niedrigen Quellungsgrad für Vergleichsbeispiele

Die Membran wurde in folgender Weise hergestellt: Als Ausgangsmaterial wurde eine, wie vorstehend genannte CA-Membran verwendet. Diese CA-Membran wurde drei Minuten bei Raumtemperatur mit 15%iger Kalilauge-Lösung in 80%igem Ethanol verseift. Anschließend wurde drei Minuten mit einer 6,8%igen Essigsäurelösung, zweimal mit Ethanol und danach 15 Minuten mit fließendem RO-(d.h. reverse osmosis)-Wasser gespült. Danach wurde die Membran 20 Minuten bei 80°C im Umlufttrockenschrank getrocknet.

Im nächsten Schritt wurde die so erhaltene, getrocknete Membran 30 Minuten bei Raumtemperatur mit 30%igem 1,4-Butandioldiglycidylether in wässriger 0,1 M Natronlauge-Lösung und wässriger 0,1%iger Natriumborhydrid-Lösung behandelt, und dann die feuchte Membran 20 Stunden in einem abgeschlossenen Gefäß bei Raumtemperatur stehen gelassen.

Abschließend wurde die Membran 30 Minuten mit fließendem Wasser gespült.

Der Wasserdurchfluß der so hergestellten, aktivierend vernetzten Cellulosehydrat-Membran betrug 630 ml/(min x bar x $cm^2$). Der Quellungsgrad betrug 1,16.

### Beispiel 2

Aktivierend vernetztes Zwischenprodukt für eine erfindungsgemäße Adsorptionsmembran

Als Ausgangsmaterial wurde eine CA-Membran wie in Beispiel 1 verwendet. Diese CA-Membran wurde 30 Minuten bei Raumtemperatur mit 0,6 M wässriger Natronlauge-Lösung (d.h. unter quellenden Bedingungen) verseift und anschließend 3 x 10 Minuten mit 0,5 M wässriger Natronlauge-Lösung gespült. Die erhaltene Membran wurde 30 Minuten bei Raumtemperatur mit 30%igem 1,4-Butandioldiglycidylether in 0,1 M wässriger Natronlauge-Lösung und 0,1%iger, wässriger Natriumborhydrid-Lösung behandelt (d.h. vernetzt), und danach wurde die feuchte Membran 20 Stunden in einem abgeschlossenen Gefäß bei Raumtemperatur stehengelassen.

Abschließend wurde 30 Minuten mit fließendem Wasser gespült.

Der Wasserdurchfluss des aktivierend vernetzten Zwischenprodukts betrug 45 ml/(min x bar x $cm^2$) und der Quellungsgrad betrug 16,2.

### Beispiel 3

Aktivierend vernetztes Zwischenprodukt für eine erfindungsgemäße Adsorptionsmembran: Vorbehandlung der CA-Membran

Eine CA-Membran wurde in gleicher Weise behandelt wie in Beispiel 2, mit der Ausnahme, daß die CA-Membran vor der Verseifung 20 Minuten bei 80°C im Trockenschrank erhitzt wurde.

Der Wasserdurchfluss des resultierenden, aktivierend vernetzten Zwischenprodukts betrug 21 ml/(min x bar x $cm^2$) und der Quellungsgrad betrug 34,8.

### Beispiel 4

Aktivierend vernetztes Zwischenprodukt für eine erfindungsgemäße Adsorptionsmembran: Vorbehandlung der CA-Membran

Eine CA-Membran wurde in gleicher Weise behandelt wie in Beispiel 2 mit der Ausnahme, daß die CA-Membran vor der Verseifung in 20%iger Essigsäure-Lösung auf 80°C erhitzt wurde und 15 Minuten mit fließendem Wasser gespült wurde.

Der Wasserdurchfluss des resultierenden, aktivierend vernetzten Zwischenprodukts betrug 180 ml/(min x bar x $cm^2$) und der Quellungsgrad betrug 4,06.

**Beispiel 5**

Verschiedene Alkalihydroxide

CA-Membranen wurden jeweils in 0,5 M wässrigen Lösungen von LiOH, NaOH und KOH 30 Minuten bei Raumtemperatur verseift, anschließend ohne Spülung mit wässrigen Lösungen von 15%igem 1,4-Butandioldiglycidylether und 0,1%iger Natriumborhydrid-Lösung in den gleichen Alkalihydroxidlösungen bei Raumtemperatur 3,5 Stunden vernetzt. Die Membranen wurden weiter mit einem quaternären Ammonium-Liganden umgesetzt, in dem die vernetzten Membranen 35 Minuten bei 30°C in einer 10%igen, wässrigen Lösung von Trimethylamin und 5 Minuten bei Raumtemperatur in 5%iger Schwefelsäure-Lösung behandelt und danach 10 Minuten mit fließendem Wasser gespült wurden. Die Ergebnisse sind in der Tabelle 2 angegeben.

**Beispiel 6**

Nichtaktivierend vernetztes Zwischenprodukt für erfindungsgemäße Adsorptionsmembran

Als Ausgangsmembran wurde eine CA-Membran wie in Beispiel 1 verwendet. Diese CA-Membran wurde 30 Minuten bei Raumtemperatur mit 0,6 M wässriger Natronlauge-Lösung verseift und anschließend 3 x 10 Minuten mit 0,5 M wässriger Natronlauge-Lösung gespült. Die erhaltene Membran wurde 30 Minuten bei Raumtemperatur mit wässrigem, 15%igen 1,4-Butandioldiglycidylether in 0,5 M wässriger Natronlauge-Lösung und 0,1%iger, wässriger Natriumborhydridlösung behandelt (vernetzt) und danach wurde die feuchte Membran 20 Stunden in einem abgeschlossenen Gefäß bei Raumtemperatur stehengelassen. Abschließend wurde 30 Minuten mit fließendem Wasser gespült.

Der Wasserdurchfluss des nicht-aktivierend vernetzten Zwischenprodukts betrug 31 ml/(min x bar x $cm^2$) und der Quellungsgrad betrug 23,5.

**Beispiel 7**

Einführung von quaternären Ammonium-Liganden (Q-Membran)

Aktivierend vernetzte Membranen (Zwischenprodukte) wurden 35 Minuten bei 30°C in einer 10%igen, wässrigen Lösung von Trimethylamin und 5 Minuten bei Raumtemperatur in 5%iger Schwefelsäure-Lösung behandelt und danach 10 Minuten mit fließendem Wasser gespült, wodurch Membranen mit quaternären Ammonium-Liganden (im folgenden: Q-Membranen) erhalten wurden.

**Beispiel 8**

Einführung von Sulfonsäure-Liganden (S-Membran)

Aktivierend vernetzte Membranen (Zwischenprodukte) wurden 45 Minuten bei 80°C in einer wässrigen Lösung von 30% Natriumsulfit und 2,5 % $Na_2HPO_4$ x $H_2O$ bei einem pH-Wert von 8,0 behandelt und danach 10 Minuten mit fließendem Wasser, 5 Minuten mit 35g einer 1%igen HCl-Lösung, 2 x 5 min mit je 30g einer wässrigen 1M NaCl-Lösung, 5 min mit 500g 5%iger $H_2SO_4$-Lösung und 10 Minuten mit fließendem Wasser gespült, wodurch Membranen mit Sulfonsäure-Liganden (S-Membranen) erhalten wurden.

**Beispiel 9**

Einführung von Iminodiessigsäure-Liganden (IDA-Membran)

Aktivierend vernetzte Membranen (Zwischenprodukte) wurden 45 Minuten bei 80°C mit einer 13%igen, wässrigen Lösung von Iminodiessigsäure bei einem pH-Wert von 11,2 behandelt und danach 10 Minuten mit fließendem Wasser, 5 Minuten mit 1%iger HCl-Lösung, 2 x 5 min mit wässriger 1 M NaCl-Lösung und 10 Minuten mit fließendem Wasser gespült, wodurch Membranen mit Iminodiessigsäure-Liganden (IDA-Membranen) erhalten wurden.

**Beispiel 10**

Einführung von Phenyl-Liganden (Ph-Membran)

Aktivierend vernetzte Membranen (Zwischenprodukte) wurden drei Stunden bei Raumtemperatur mit einer wässrigen Lösung von 1% Anilin in 0,1 M Kpi (Kaliumphosphat)-Puffer bei einem pH-Wert von 8,0 behandelt und die feuchten Proben wurden 19 Stunden im verschlossenen Gefäß belassen. Nach 15 Minuten Spülen mit fließendem Wasser wurde 15 Minuten mit 1 M wässriger NaCl-Lösung und 15 Minuten mit fließendem Wasser nachgespült, wodurch Membranen mit Phenyl-Liganden (Ph-Membranen) erhalten wurden.

**Beispiel 11**

Einführung von p-Aminobenzamidin-Liganden (pABA-Membran)

Eine nicht-aktivierend vernetzte Cellulosehydrat-Membran (Zwischenprodukt) nach Beispiel 6 wurde durch 30minütige Behandlung mit einer 1%igen wässrigen Lösung von Natriumperiodat bei Raumtemperatur aktiviert, 15 Minuten mit fließendem Wasser gespült, eine Stunde bei Raumtemperatur mit einer auf einen pH-Wert von 5,6 eingestellten Lösung von

4,3 g p-Aminobenzamidin-Dihydrochlorid,
2,17g Natrium-Cyanoborhydrid,
2g 1 M Natronlauge, und
34,8g McIlvaine-Puffer mit einem pH-Wert von 5,6 (Gemisch von 0,1 M Citronensäure Monohydrat (Riedel-de-Haen Cat. 33114) und 0,2 M Dinatriumhydrogenphosphat-dihydrat (Merck Cat. 1.06580). 21g Citronensäure Monohydrat in 1l "Reverse-Osmosis"-Wasser (ROW) lösen = 0,1 M. 35,6g Di-Natriumhydrogenphosphat-dihydrat in 1l ROW lösen = 0,2M. 500g 0,2 M Di-Natriumhydrogenphosphat-dihydrat vorlegen und mit 0,1 M Citronsäure Monohydrat pH-Wert auf 5,6 einstellen)

behandelt, 15 Minuten mit fließendem Wasser gespült, nacheinander mit 100g 1%iger wässriger $NaBH_4$-Lösung und 100g 1 M wässriger NaCl-Lösung behandelt und nochmals 15 Minuten mit fließendem Wasser gespült. Dadurch wurden Membranen mit p-Aminobenzamidin-Liganden (pABA-Membranen) erhalten.

**Beispiel 12**

Einführung von Cibacronblau-3GA-Liganden (CB Membran)

Eine nicht-aktivierend vernetzte Cellulosehydrat-Membran (Zwischenprodukt) nach Beispiel 6 wurde 24 Stunden bei Raumtemperatur mit einer Lösung, hergestellt durch Versetzen einer 10 Minuten bei 80°C gerührten und bei Raumtemperatur mit 3%iger wässriger Natronlauge-Lösung versetzten 2%igen wässrigen Lösung des Farbstoffs Cibacronblau-3GA behandelt und nacheinander 60 Minuten mit fließendem Wasser, viermal je dreißig Minuten mit Wasser bei 80°C und 15 Minuten mit fließendem Wasser gespült. Dadurch wurden Membranen mit Cibacronblau-3GA-Liganden (CB-Membranen) erhalten.

**Beispiel 13**

Trocknung der erfindungsgemäßen Adsorptionsmembran

CA-Membranen wurden in 0,5 M wässriger Natronlauge-Lösung 30 Minuten bei Raumtemperatur verseift, anschließend ohne Spülung mit einer Lösung von 30%igem 1,4-Butandioldiglycidylether und 0,1%igem Natriumborhydrid in 0,5 M wässriger Natronlauge-Lösung bei Raumtemperatur 2,5 Stunden vernetzt, danach mit Trimethylamin derivatisiert und sowohl im ungetrockneten Zustand als auch im bei 80°C im Umlufttrockenschrank getrockneten Zustand hinsichtlich ihrer statischen Bindungskapazität untersucht. Die Ergebnisse sind in der Tabelle 2 angegeben.

**Beispiel 14**

Bestimmung des Anteils an Ultraporen am Gesamtporenvolumen der Membranen

Für die Bestimmung des Anteils an Ultraporen am Gesamtvolumen der Membranen wurde die CA-Membran bei unterschiedlichen Natronlaugekonzentrationen analog zu Beispiel 2 verseift, mit 0,5M NaOH gespült, wie im Beispiel 2 vernetzt und mit Sulfonsäureliganden wie im Beispiel 8 modifiziert (Membranen 1-6). Zum Vergleich wurden die Membran aus Beispiel 1 (Membran A) und die im Stand der Technik bekannten Mikrofiltrationsmembranen der Fa. Sartorius Stedim Biotech GmbH mit Porengrößen im Bereich 0,2-0,45μm (Membranen B-F) verwendet.

Als Dextranblau wurde im Handel erhältliches Dextran aus Leuconostoc mesenteroides, Strain B 512, modifiziert mit Reactive Blue 2 dye, ca. 0,1 mmol Reactive Blue 2 pro Gramm Dextran (Blue Dextran Molecular Weight (Mw) 2.000.000 von Sigma, St. Louis, Missouri, USA, Produktnummer D 5751, CAS-Nummer: 87915-38-6), verwendet.

Der hydrodynamische Durchmesser d dieses Dextranblaus kann mit Hilfe der Gleichung nach Mark-Houwink-Sakurada:

$$d[nm]=0{,}054 \times Mw^{0,5}$$

berechnet werden und beträgt 76,4 nm.

Ultraporen sind, wie vorstehend definiert, Poren, die für Dextranblau nicht zugänglich sind.

Das für Wasser zugängliche Porenvolumen wird als Vw [$cm^3$] bezeichnet. Es wird angenommen, daß alle Membranporen für Wasser zugänglich sind, so daß Vw dem Gesamtporenvolumen der Membran entspricht.

Das für Dextranblau zugängliche Porenvolumen wird als Vd [$cm^3$] bezeichnet.

Das für Dextranblau nicht zugängliche Porenvolumen der Ultraporen wird als Vp [$cm^3$] bezeichnet.

Vp wird durch das erfindungsgemäße Verfahren, bei dem die Celluloseester-Membran während der Verseifung quillt, vergrößert.

Es gilt

$$Vw = Vd + Vp \text{ und } Vp = Vw - Vd$$

Der prozentuale Anteil von für Dextranblau unzugänglichen Poren in der Membran ist

$$\%\ Vp = 100 \times (Vw - Vd)/Vw.$$

Das für Dextranblau zugängliche Porenvolumen Vd wird nach folgendem Verfahren bestimmt:

10 ml einer Lösung von Dextranblau in RO-Wasser bekannter Konzentration (c0) wird durch eine nasse Membran filtriert. Dadurch wird das Wasser aus dem für Dextranblau zugänglichen Porenvolumen mit der Dextranblau-Lösung ausgetauscht. Die Voraussetzung für die Methode ist, daß die Membran das Dextranblau nicht adsorptiv bindet. Dies ist für unmodifizierte Cellulosehydrat-Membranen, vernetzt und unvernetzt, gegeben. Eine Membran mit einem Durchmesser von 50 mm (d.h. einer Fläche von 19,6 $cm^2$) wird mit fließendem RO-Wasser 15 Minuten intensiv gewaschen. Die nasse Membran wird dann in ein Filtrationsgehäuse eingebaut und 10 ml Dextranblau-Lösung mit einer Konzentration von 5 mg/ml (c0) werden bei 0,1 bar Druck durch die Membran filtriert. Dann wird die Membran aus dem Filtrationsgehäuse ausgebaut, ein Stanzling mit einem Durchmesser von 47 mm (d.h. einer Fläche von 17,3 $cm^2$) wird mittig ausgestanzt (um die abgedichteten Ränder der Membran zu entfernen) und mit einem Labortuch (Kimtech Science, 200, 2, 21 x 20 cm, weiß, 7101) abgetupft.

Danach wird die Membran in einer genau bestimmten Menge (Volumen V = 5,0 ml) RO-Wasser in einem abgedichteten Gefäß 20 Stunden bei 80 Upm geschüttelt. Die Konzentration der Dextranblau-Lösung (c) wird dann photometrisch bei 618 nm bestimmt. Der Extinktionskoeffizient E (1 mg/ml; 1 cm) der Dextranblau-Lösung beträgt 0,896. Aus der Konzentration der Dextranblau-Lösung wird das für Dextranblau zugängliche Porenvolumen berechnet:

$$Vd\ [cm^3] = c \times V/c0$$

Das für Wasser zugängliche Porenvolumen wird nach folgendem Verfahren bestimmt:

Die Membranprobe wird mit fließendem RO-Wasser 15 Minuten intensiv gewaschen. Das an der Membran anhaftende Wasser wird mit dem Labortuch abgetupft und die nasse Membran wird gewogen. Danach wird die Membran bei 80°C in einem Umlufttrockenschrank 30 Minuten getrocknet und die getrocknete Membran wird gewogen. Die Gewichtsdifferenz zwischen nasser und trockener Membran entspricht der Wassermenge in der Membran (Vw). Dabei wird eine Wasserdichte von 1,0g/cm$^3$ angenommen.

$$\text{Aus } \% \ Vp = 100 \times (Vw - Vd)/Vw$$

wird der prozentuale Anteil des für Dextranblau nicht zugänglichen Porenvolumens am Gesamtporenvolumen berechnet.

Mit steigender Natronlaugekonzentration bei der Verseifung der Celluloseacetat-Membran wird die Quellung stärker, der Quellungsgrad steigt, die Permeabilität der Membran sinkt, die Membrandicke nimmt zu, der Anteil an Ultraporen am Gesamtporenvolumen nimmt zu und die Bindungskapazität steigt, wie aus der folgenden Tabelle 1 ersichtlich ist.

Tabelle 1

| | Verseifung c (NaOH) [M] | Spülung nach Verseifung c(NaOH) [M] | Vp [%] | Permeabilität 10mM Kpi pH7 S Membran [ml/(min x bar x cm$^2$)] | Bindungskapazität Lysozym S Membran [mg/cm$^2$] | Quellungsgrad [-] |
|---|---|---|---|---|---|---|
| 1 | 0,20 | 0,5 | 16% | 515 | 0,75 | 1,4 |
| 2 | 0,40 | 0,5 | 25% | 341 | 1,40 | 2,1 |
| 3 | 0,50 | 0,5 | 30% | 180 | 1,71 | 4,1 |
| 4 | 0,60 | 0,5 | 34% | 73 | 1,99 | 10,0 |
| 5 | 0,75 | 0,5 | 39% | 8 | 2,23 | 90,1 |
| 6 | 1,00 | 0,5 | 45% | 4 | 2,40 | 208,6 |

**Auswertung der Membranen (für die Beispiele 1 bis 14 und die Vergleichsbeispiele 1 und 2)**

Die Auswertung der erhaltenen Membranen erfolgte in der nachstehend beschriebenen Weise:

1) Durchflussbestimmung
Membranen mit einer aktiven Membranfläche von 12,5 cm$^2$ wurden jeweils in ein Gehäuse eingebaut und es wurde die Zeit für die Filtration von 100 ml Wasser oder Puffer gemessen. Die in der Tabelle 2 wiedergegebenen Durchflussangaben für mit funktionellen Gruppen umgesetzten Membranen beziehen sich auf den entsprechenden Bindungspuffer. Es wurden die gleichen Puffer verwendet wie für die unten beschriebenen Bestimmungen der Bindungskapazitäten.

2) Bestimmung der statischen Bindungskapazität von Q-Membranen Membranproben mit einer aktiven Membranfläche von jeweils 17,6 cm$^2$ wurden in 35 ml 20 mM TRIS/HCl pH 7,3 3 x 5 min mit etwa 80 Umdrehungen pro Minute (Upm) geschüttelt. Danach wurden 35 ml einer Lösung von 2 mg/ml Rinderserumalbumin (RSA)-Lösung in 20 mM TRIS/HCl PH 7,3 12-18 Stunden bei 20-25°C mit etwa 80 Upm geschüttelt. Anschließend wurden die Membranproben 2 x 15 Minuten in jeweils 35 ml 20mM TRIS/HCl pH 7,3 gespült. Danach wurden die Membranproben in 20 ml 20 mM TRIS/HCl pH 7,3 + 1 M wässriger NaCl-Lösung geschüttelt. Die Menge des eluierten Proteins wurde durch Messung der optischen Dichte (OD) bei 280 nm bestimmt.

3) Bestimmung der statischen Bindungskapazität von S-Membranen
Membranproben mit einer aktiven Membranfläche von jeweils 17,6 cm$^2$ wurden in 35 ml 10 mM KPi pH 7,0 3 mal 5 Minuten mit etwa 80 Upm geschüttelt. Danach wurden 35 ml einer Lösung von 2 mg/ml Lysozym in 10 mM KPi pH 7,6 12-18 Stunden bei 20 - 25°C mit etwa 80 Upm geschüttelt. Anschließend wurden die Membranproben 2 x 15 Minuten in jeweils 35 ml 10 mM KPi pH 7,0 gespült. Danach wurden die Membranproben in 20 ml 10 mM KPi pH 7,0 + 1 M wässriger NaCl-Lösung geschüttelt. Die Menge des eluierten Proteins wurde durch Messung der optischen Dichte (OD) bei 280 nm bestimmt.

4) Bestimmung der statischen Bindungskapazität von IDA-Membranen
Membranproben mit einer aktiven Membranfläche von 17,6 cm$^2$ wurden in 35 ml 10 mM KPi pH 7,0 3 x 5 Minuten mit etwa 80 Upm geschüttelt. Danach wurden 35 ml einer Lösung von 2 mg/ml Lysozym in 10 mM KPi pH 7,0 12-18 Stunden bei 20 bis 25°C mit etwa 80 Upm geschüttelt. Anschließend wurden die Membranproben 2 x 15 Minuten in jeweils 35 ml 10 mM KPi pH 7,0 gespült. Danach wurden die Membranproben in 20 ml 10 mM KPi pH 7,0 + 1 M wässrige NaCl-Lösung geschüttelt. Die Menge des eluierten Proteins wurde durch Messung der optischen Dichte (OD) bei 280 nm bestimmt.

5) Bestimmung der statischen Bindungskapazität von Metallchelat-Membranen (Iminodiessigsäureligand (IDA) komplexiert mit $Cu^{2+}$-Kationen)
IDA-Membranproben mit einer aktiven Membranfläche von 3,1 $cm^2$ wurden in einen Polycarbonatvorsatz eingespannt und an eine Schlauchpumpe angeschlossen. Jeweils 10 ml Lösungen in folgender Reihenfolge wurden mit einer Flußgeschwindigkeit von 2 ml/min mit Hilfe der Schlauchpumpe durch die Membranen gepumpt:

1. 0,1 M $CH_3COONa$ + 0,5 M NaCl pH 4,5
2. 0,1 M $CH_3COONa$ + 0,5 M NaCl pH 4,5 + 0,1 M $CuSO_4$
3. 0,1 M $CH_3COONa$ + 0,5 M NaCl pH 4,5
4. 0,05 M KPi + 0,5 M NaCl pH 7,5
5. 2 mg/ml Cytochrom C in 0,05 M KPi + 0,5 M NaCl pH 7,5
6. 0,05 M KPi + 0,5 M NaCl pH 7,5
7. 0,1 M Imidazol in 0,05 M KPi + 0,5 M NaCl pH 7,5
8. 1 M $H_2SO_4$

Die Menge des eluierten Proteins in Schritt 7 wurde durch Messung der optischen Dichte (OD) bei 528 nm bestimmt.

6) Bestimmung der statischen Bindungskapazität von Ph-Membranen
Membranproben mit einer aktiven Membranfläche von 3,1 $cm^2$ wurden in einen Polycarbonatvorsatz eingespannt und an eine Schlauchpumpe angeschlossen. Lösungen in folgender Reihenfolge wurden mit einer Flußgeschwindigkeit von 2 ml/min mit Hilfe der Schlauchpumpe durch die Membranen gepumpt:

1. 10 ml 0,05 M KPi + 1M $(NH_4)_2$ $SO_4$ NaCl pH 7,0
2. 20 ml 1 mg/ml gamma-Globulin in 0,05 M KPi + 1M $(NH_4)_2SO_4$ NaCl pH 7,0
3. 20 ml 0,05 M KPi + 1M $(NH_4)_2$ $SO_4$ NaCl pH 7,0
4. 10 ml 0,05 M KPi pH 7,0

Die Menge des eluierten Proteins in Schritt 4 wurde durch Messung der optischen Dichte (OD) bei 280 nm bestimmt.

7) Bestimmung der statischen Bindungskapazität von pABA Membranen
Membranproben mit einer aktiven Membranfläche von 3,1 $cm^2$ wurden in einen Polycarbonatvorsatz eingespannt und an eine Schlauchpumpe angeschlossen. Lösungen in folgender Reihenfolge wurden mit einer Flußgeschwindigkeit von 2 ml/min mit Hilfe der Schlauchpumpe durch die Membranen gepumpt:

1. 10 ml 50 mM TRIS/HCl pH 8,8 + 10 mM $CaCl_2$ + 250 mM NaCl
2. 10 ml 2 mg/ml Trypsin Type I in 50 mM TRIS/HCl pH 8,8 + 10 mM $CaCl_2$ + 250 mM NaCl
3. 10 ml 50 mM TRIS/HCl pH 8,8 + 10 mM $CaCl_2$ + 250 mM NaCl
4. 10 ml 0,1 M Glycin/HCl pH 2,8

Die Menge des eluierten Trypsins im Schritt 4 wurde durch Trypsinbestimmung nach Bergmeyer gemäß folgendem Verfahren bestimmt. Die enzymatische Aktivität von Trypsin wird durch Verschiebung der Extinktion von N-$\alpha$-Benzoyl-L-argininethylesterhydrochlorid (BAEE) bei einer Wellenlänge von 253 nm bei der durch Trypsin katalysierten Hydrolyse als $\Delta E$/min bestimmt.
In einer Halbmicroquarzküvette werden die folgenden Lösungen in der angegebenen Reihenfolge gemischt:

1. 850$\mu$l Puffer (50 mM TRIS/HCl pH8,8 + 10mM $CaCl_2$ + 250mM NaCl),
2. 100$\mu$l BAEE-Lösung in Bindungspuffer (Sigma Best.Nr. B 4500) und
3. 501$\mu$l Probe.

Die gefüllte Küvette wird im Photometer platziert und nach 5 s wird $\Delta$E/min bei 253 nm bestimmt.

8) Bestimmung der statischen Bindungskapazität von CB-Membranen Membranproben mit einer aktiven Membranfläche von 9,8 $cm^2$ wurden in 10 ml 0,1 M wässriger Natronlauge-Lösung 10 Minuten geschüttelt und dann in 10 ml 10 mM KPi pH 7,3 3 x 10 Minuten mit etwa 80 Upm geschüttelt. Danach wurden 5 ml einer Lösung von 2 mg/ml Rinderserumalbumin (RSA) in 10 mM KPi pH 7,0 12-18 Stunden bei 20 - 35°C mit etwa 80 Upm geschüttelt. Anschließend wurden die Membranproben 3 x 10 Minuten in jeweils 10 ml 10 mM KPi pH 7,0 gespült. Danach wurden die Membranproben eine Stunde in 5 ml 10 mM KPi pH 7,0 + 1 M wässriger NaCl-Lösung geschüttelt. Die Menge des eluierten Proteins wurde durch Messung der optischen Dichte (OD) bei 280 mm bestimmt.

9) Konfokale Laserfluoreszenzmikroskopie

Markierung der Membranen

Eine Adsorptionsmembran aus Beispiel 1 und eine erfindungsgemäße Adsorptionsmembran nach Beispiel 2 wurden nach Beispiel 8 mit Sulfonsäureliganden versehen. Zusammen mit einer unter der Bezeichnung Sartobind® S im Handel erhältlichen Adsorptionsmembran der Sartorius Stedim Biotech GmbH mit einem mit Sulfonsäure-belegten Hilfspolymer wurden die Membranen mit dem OH-reaktiven Fluoreszenzfarbstoff "5-DTAF" (5-(4,6-Dichlorotriazinyl)aminofluorescein, Anregungs- bzw. Emissionswellenlänge von 492 nm bzw. 516 nm, Firma Invitrogen) markiert. Die Inkubation der Lösung des Farbstoffs sowie alle folgenden Waschschritte wurden mit jeweils drei Membranproben (Durchmesser: 13 mm) in einem Filterhalter bei kontinuierlicher Durchspülung mit einem Durchfluß von ca. 1 ml/min durchgeführt. Dabei wurden jeweils 20 ml einer 5-DTAF-Lösung in 100 mM Natriumhydrogencarbonat-Lösung mit einem pH-Wert von 9,3 mit an die Membran angepaßten Konzentrationen, nämlich 13,5 $\mu$g/ml 5-DTAF + 100 mM NaCl-Lösung für die Membranen nach den Beispielen 1 und 2 und 25 $\mu$g/ml 5-DTAF + 200 mM NaCl-Lösung für die Sartobind® S Membran, verwendet. Da vermutet wurde, daß die dreidimensionale Kationenaustauscherschicht eine besonders effektive Abschirmung der Cellulosematrix bewirkt, wurde für die Sartobind® S Membran sowohl die Farbstoffals auch die Salzkonzentration erhöht. Nach Durchspülung für ca. 18 Stunden wurden die Proben anschließend nacheinander mit jeweils 100 ml einer 20%igen Ethanollösung, 1M NaCl-Lösung und 200 mM Natriumphosphat-Puffer, pH 7,0, gewaschen. Für die CLSM-Analyse wurde wegen der besten Homogenität der Markierung jeweils die zweite Probe im Filterhalter benutzt.

Markierung und Reinigung des Proteins

Lysozym (erhältlich von der Firma Sigma, St. Louis, Missouri, USA; Protein ca. 95%, ca. 50 000 units/mg Protein) wurde mit dem $NH_2$-reaktiven Fluoreszenzfarbstoff "Cy5 mono-reactive NHS Ester" (erhältlich von der Firma GE Health Care Bio-Sciences AB, Uppsala, Schweden) in Natriumcarbonat-Puffer, pH 9,3, markiert sowie anschließend zunächst durch Gelfiltration und dann durch HPIonenaustauschchromatographie gereinigt. Durch entsprechende Auswahl der Fraktionen der Chromatographie wurde das einfach markierte Lysozym in reiner Form erhalten. Danach erfolgte mittels Ultrafiltration die Aufkonzentrierung auf die für das Bindungsexperiment notwendige Konzentration. Die Bestimmung der Konzentration des markierten Lysozyms erfolgte mittels UV-Vis-Photometer (Messung der Absorptionen bei 280 nm und 650 nm).

Inkubation der Membranen mit Protein

Proben der mit 5-DTAF-markierten Membranen wurden mit einem Durchmesser von 5 mm ausgestanzt und für vier Stunden in einer Lösung des markierten Lysozyms mit einer Konzentration von 0,6 g/L in 200 mM Natriumphosphat-Puffer, pH 7,0 + 50 mM wässrige NaCl-Lösung inkubiert (für 1 $cm^2$ Proben wurden jeweils 4,1 ml Proteinlösung eingesetzt). Danach wurden die Proben mit dem Puffer für 15 Minuten gewaschen.

CLSM Analyse

Die Analyse erfolgte mit dem CLSM-System Leica TCS SP. Jede Probe wurde in 200 mM Natriumphosphat-Puffer von beiden Oberflächen aus untersucht. Zunächst erfolgte die Bestimmung der geeigneten Signalverstärkung (Kriterien: unterdrückte Autofluoreszenz der Membran; das Maximum der Signalverstärkung wurde mit Hilfe des Histogramms in der Auswertesoftware "Zeiss LSM Image Browser" eingestellt, um örtliche Überbelichtungen zu vermeiden) und Identifizierung von z = 0 (Kriterien: hohe Streuintensität und anschließende erstmalige Identifizierung der Porenmorphologie bei weiterer Verringerung des Abstands zur Probe). Danach wurde in x,y-Scans an verschiedenen z-Positionen die aus SEM bekannte charakteristische Morphologie der Sartobind® S Membranen gesucht. Danach erfolgten in einem engen Bereich verschiedener z-Positionen (in ca. 20 Mikrometer Tiefe, in Abständen von 1 Mikrometer in beide Richtungen) detaillierte x, y-Scans der beiden Anregungswellenlängen (488 nm für 5-DTAF, 633 nm für Cy5). Für jede Probe und jede Orientierung wurden diese Scans jeweils für drei verschiedene Positionen durchgeführt. Die Probe Sartobind® S wurde zuerst vermessen; die für diese Probe gewählten Einstellungen (z-Position und Signalverstärkung) wurden bei der Messung der anderen Membranproben beibehalten. Weil die Signalintensitäten von der erfindungsgemäßen Membran nach Beispiel 2 bei 633 nm sehr viel höher als bei den anderen beiden Membranen waren, wurde eine Verringerung der Signalverstärkung vorgenommen:

"Gains" (488 nm/633nm)
Membran Sartobind® S: 426/643
Membran nach Beispiel 1: 426/669
Membran nach Beispiel 2: 357/650

CLSM Auswertung

Die Auswertungen erfolgten mit Hilfe des Zeiss LSM Image Browsers 3.5.0.376. Aus den erhaltenen Bildern wurden detaillierte x,y-Scans in einem Bereich der z-Positionen von ca. 20 $\mu$m Tiefe für die Oberseite ausgewählt. Die erhaltenen Bilder wurden jeweils als 8-Bit Bilder mit einer Auflösung von 512 x 512 Pixel, entsprechend 146,2 x 146,2 $\mu m^2$, dargestellt. Die Figuren 2 bis 4 zeigen die Überlappung der beiden Bilder der Verteilung von Lysozym und der Porenmorphologie der Cellulose. Zusätzlich wird für jede Messung am oberen und rechten Rand der Darstellung auch noch ein Intensitätsprofil der Intensitäten für beide Fluoreszenzmarkierungen gezeigt.

Ergebnisse der Versuche

Die Ergebnisse der Versuche sind in der nachstehenden Tabelle 2 gezeigt.

Tabelle 2

| Membran aus Beispiel | Bemerkung | Ligand | Protein | Durchfluss [ml/(min x bar x cm$^2$)] | Bindungskapazität [mg/cm$^2$] |
|---|---|---|---|---|---|
| 1 | | Q | RSA | 643 | 0,07 |
| | | S | Lysozym | 664 | 0,01 |
| | | IDA | Lysozym | 681 | 0,03 |
| | | IDA+Cu2+[1)] | Cytochrom C | 681 | 0,15 |
| | | Ph | gamma-Globulin | 570 | 0,2 |
| 2 | | Q | RSA | 44 | 0,92 |
| | | S | Lysozym | 38 | 2,06 |
| | | IDA | Lysozym | 41 | 1,91 |
| | | IDA+Cu2+[1)] | Cytochrom C | 41 | 0,5 |
| | | Ph | gamma-Globulin | 31 | 1,26 |
| 3 | | Q | RSA | 20 | 1,13 |
| | | S | Lysozym | 24 | 2,85 |
| 4 | | Q | RSA | 158 | 0,74 |
| | | S | Lysozym | 167 | 3,11 |
| | | S | gamma-Globulin | 167 | 0,44 |
| 5 | LiOH | Q | RSA | 70 | 1,18 |
| | NaOH | Q | RSA | 109 | 0,93 |
| | KOH | Q | RSA | 519 | 0,08 |
| 6 | | CB | RSA | 30 | 0,31 |
| | | pABA | Trypsin | 35 | 0,75 |
| 13 | ungetrocknet | Q | RSA | 213 | 0,94 |

(fortgesetzt)

| Membran aus Beispiel | Bemerkung | Ligand | Protein | Durchfluss [ml/(min x bar x cm$^2$)] | Bindungskapazität [mg/cm$^2$] |
|---|---|---|---|---|---|
| | getrocknet | Q | RSA | 239 | 0,92 |
| [1)] Metallchelat aus Iminodiessigsäureligand (IDA) komplexiert mit $Cu^{2+}$-Kationen | | | | | |

**Vergleichsbeispiel 1**

Gleichzeitige Verseifung und Vernetzung wie im Beispiel 1, Probe K10C der WO 2007/017085 A2, aber mit 1,4-Butandioldiglycidylether anstelle von Epichlorhydrin, unter nicht-quellenden Bedingungen.

Es wurden eine wie vorstehend definierte CA-Membran und eine 0,65 $\mu$m Celluloseacetat-Membran wie in Bsp. 1, Probe K10C der WO 2007/017085 A2 mit einem Wasserdurchfluß von 65 ml/(min x bar x cm$^2$) als Ausgangsmembranen verwendet.

Die Celluloseacetatmembranen wurden in 100g Wasser, 10g $Na_2SO_4$ und 1g 1,4-Butandioldiglycidylether auf 47°C erhitzt und 10g einer 1M wässrigen Natronlauge-Lösung wurden während 30 Minuten zudosiert. Die Membranen wurden weiter in der Lösung 3,5 Stunden bei 47°C behandelt und anschließend 30 Minuten mit fließendem Wasser gespült. In die Membranen wurden quaternäre Ammonium-Liganden eingeführt, wobei Q-Membranen erhalten wurden. Die aus der CA-Membran erhaltene, verseifte und vernetzte Q-Membran wies einen Wasserdurchfluss von 589 ml/(min x bar x cm$^2$), einen Quellungsgrad von 1,2 und eine Bindungskapazität für RSA von 0,04 mg/cm$^2$ auf. Die aus der Celluloseacetat-Membran gemäß Beispiel 1, Probe K10C der WO 2007/017085 A2 erhaltene, verseifte und vernetzte Q-Membran wies einen Wasserdurchfluss von 66 ml/(min x bar x cm$^2$), einen Quellungsgrad von 1,0 und eine Bindungskapazität für RSA von 0,04 mg/cm$^2$ auf.

**Vergleichsbeispiel 2** Versuch zur Quellung von bereits verseiften Cellulosehydrat-Membranen

Eine als Ausgangsmembran verwendete und wie vorstehend definierte CA-Membran wurde drei Minuten bei Raumtemperatur mit einer 15%igen Kalilauge-Lösung in 80%igem Ethanol verseift und anschließend drei Minuten mit einer 6,8%igen Essigsäure-Lösung, zweimal mit Ethanol und 15 Minuten mit fließendem RO-Wasser gespült. Die erhaltene, verseifte Membran wurde 30 Minuten bei Raumtemperatur mit einer 0,6 M wässrigen Natronlauge behandelt und danach dreimal 10 Minuten mit einer 0,5 M wässrigen Natronlauge gespült. Anschließend wurde die Membran 30 Minuten bei Raumtemperatur mit einer 30%igen Lösung von 1,4-Butandioldiglycidylether in 0,1 M wässriger Natronlauge-Lösung und 0,1%iger, wässriger Natriumborhydrid-Lösung behandelt, worauf die feuchte Membran 20 Stunden in einem abgeschlossenen Gefäß bei Raumtemperatur stehengelassen wurde. Schließlich wurde die erhaltene Membran 30 Minuten mit fließendem Wasser gespült.

Der Wasserdurchfluss der so hergestellten, verseiften und vernetzten Cellulosehydrat-Membran betrug 688 ml/(min x bar x cm$^2$) und der Quellungsgrad betrug 1,06.

In zwei Proben der Membran wurden, wie in den Beispielen 7 bzw. 9 beschrieben, quaternäre Ammoniumliganden bzw. Sulfonsäureliganden eingeführt, wodurch eine Q-Membran und eine S-Membran erhalten wurden. Die Q-Membran wies eine Bindungskapazität für RSA von 0,044 mg/cm$^2$ auf und die S-Membran eine Bindungskapazität für Lysozym von 0,067 mg/cm$^2$.

**Beispiele für die erfindungsgemäßen Membranen mit Funktionalisierung durch Polyamine:**

**Beispiel 15**

Funktionalisierung durch Polyethylenimin:

Die CA-Membran wurde 30 Minuten bei Raumtemperatur mit 0,6 M wässriger Natronlauge-Lösung (d.h. unter quellenden* Bedingungen) verseift und anschließend 3 x 10 Minuten mit 0,5 M wässriger Natronlauge-Lösung gespült. Die erhaltene Membran wurde 30 Minuten bei Raumtemperatur mit 30%igem 1,4-Butandioldiglycidylether in 0,1 M wässriger Natronlauge-Lösung und 0,1%iger wässriger Natriumborhydrid-Lösung behandelt (d.h. vernetzt und mit reaktionsfähigen Epoxygruppen versehen) und danach wurde die feuchte Membran 20 Stunden in einem abgeschlossenen Gefäß bei Raumtemperatur stehengelassen. Abschließend wurde 30 Minuten mit fließendem Wasser gespült.

500 cm$^2$ Membran wurden in jeweils 500 g 50%-iger Lösung von Lupasol® FG (Polyethylenimin (PEI) der BASF AG, CAS-Nummer 25987-06-8, Molmasse 800 g/mol) in RO-Wasser (Reverse-Osmose-Wasser) 1 h bei 50 °C behandelt. Die Membranen wurden anschließend 5 Minuten bei Raumtemperatur mit 5%iger Schwefelsäure-Lösung behandelt und danach 10 Minuten mit fließendem Wasser gespült.

**[0171]** * Die Quellung der Celluloseester-Matrix während der Hydrolyse (Verseifung) der Estergruppen wird durch den Quellungsgrad, d.h. das Verhältnis der Wasserpermeabilität der zuvor mit Wasser benetzten Celluloseester-Membran zur Wasserpermeabilität der endgültigen, d.h. verseiften, gegebenenfalls aktivierend vernetzten und mit Ligand(en) versehenen Cellulosehydrat-Membran, beschrieben.

**Beispiel 16**

Funktionalisierung durch Polyethylenimin

Die CA-Membran wurde wie im Beispiel 15 verseift, vernetzt und mit reaktionsfähigen Epoxygruppen versehen. 500 cm$^2$ Membran wurden in jeweils 500 g 30%-iger Lösung von Lupasol® WF (Polyethylenimin der BASF AG, Molmasse 25.000 g/mol) in RO-Wasser 1 h bei 50 °C behandelt. Die Membranen wurden anschließend 5 Minuten bei Raumtemperatur mit 5%iger Schwefelsäure-Lösung behandelt und danach 10 Minuten mit fließendem Wasser gespült.

**Beispiel 17**

Funktionalisierung durch Polyethylenimin

Die CA-Membran wurde wie im Beispiel 15 verseift, vernetzt und mit reaktionsfähigen Epoxygruppen versehen. 500 cm$^2$ Membran wurden in jeweils 500 g 20%-iger Lösung von Lupasol® PN 50 (Polyethylenimin der BASF AG, Molmasse 1.000.000 g/mol) in RO-Wasser 1 h bei 50 °C behandelt. Die Membranen wurden anschließend 5 Minuten bei Raumtemperatur mit 5%iger Schwefelsäure-Lösung behandelt und danach 10 Minuten mit fließendem Wasser gespült.

**Beispiel 18**

Funktionalisierung durch Polyethylenimin, immobilisiert über Aldehydgruppen

Die CA-Membran wurde wie im Beispiel 15 verseift und aktiviert. Danach wurde die Membran mit 5%iger Schwefelsäure-Lösung 3 h bei 50 °C behandelt. Anschließend wurde die Membran 30 min mit 1%-iger wässriger $NaIO_4$-Lösung behandelt und 15 min mit fließendem RO-Wasser gespült. 500 cm$^2$ Membran wurden mit 500 g einer Lösung von 20 % Lupasol® WF (BASF AG, Molmasse 25.000 g/mol) und 0,3 % $NaBH_4$ in RO-Wasser 2 h bei 22 °C behandelt. Die Membran wurde weiter 15 min mit 1 % $NaBH_4$ in RO-Wasser behandelt, um restliche Aldehydgruppen zu reduzieren. Weiter wurde die Membran 15 min mit fließendem RO-Wasser gespült, 5 Minuten bei Raumtemperatur mit 5%iger Schwefelsäure-Lösung behandelt und danach 10 Minuten mit fließendem Wasser gespült.

**Beispiel 19**

Funktionalisierung durch Polyvinylamin

Die CA-Membran wurde wie im Beispiel 15 verseift, vernetzt und mit reaktionsfähigen Epoxygruppen versehen. 500 cm$^2$ Membran wurden in 500 g 10%-iger Lösung von Lupamin® 1595 (Polyvinylamin der BASF AG, Molmasse ~10.000 g/mol) in einer 32gewichtsprozentigen Lösung von NaOH in RO-Wasser bei pH 12,0 1 h bei 50 °C behandelt. Die Membranen wurden anschließend 5 Minuten bei Raumtemperatur mit 5%iger Schwefelsäure-Lösung behandelt und danach 10 Minuten mit fließendem Wasser gespült.

**Beispiel 20**

Funktionalisierung durch Polyvinylamin

Die CA-Membran wurde wie im Beispiel 15 verseift, vernetzt und mit reaktionsfähigen Epoxygruppen versehen. 500 cm$^2$ Membran wurden in 500 g 5%-iger Lösung von Lupamin® 9095 (Polyvinylamin der BASF AG, Molmasse ~430.000 g/mol) in einer 32gewichtsprozentigen Lösung von NaOH in RO-Wasser bei pH 12,0 1 h bei 50 °C behandelt. Die Membranen wurden anschließend 5 Minuten bei Raumtemperatur mit 5%iger Schwefelsäure-Lösung behandelt und danach 10 Minuten mit fließendem Wasser gespült.

**Beispiel 21**

Funktionalisierung durch Polyallylamin

Die CA-Membran wurde wie im Beispiel 15 verseift und aktiviert. 500 $cm^2$ Membran wurden mit 500 g einer 20%-igen Lösung von Polyallylamin (Fa. Nitto Boseki, Molmasse 15.000 g/mol) in RO-Wasser 1 h bei 50 °C behandelt. Die Membranen wurden anschließend 5 Minuten bei Raumtemperatur mit 5%iger Schwefelsäure-Lösung behandelt und danach 10 Minuten mit fließendem Wasser gespült.

**Beispiel 22**

Funktionalisierung durch Polyallylamin

Die CA-Membran wurde wie im Beispiel 15 verseift und aktiviert. 500 $cm^2$ Membran wurden mit 500 g einer 20%-igen Lösung von Polyallylamin (Fa. Nitto Boseki, Molmasse 150.000 g/mol) in RO-Wasser 1 h bei 50 °C behandelt. Die Membranen wurden anschließend 5 Minuten bei Raumtemperatur mit 5%iger Schwefelsäure-Lösung behandelt und danach 10 Minuten mit fließendem Wasser gespült.

**Beispiel 23**

Funktionalisierung durch Polyethylenimin mit hydrophoben Funktionalitäten

Die Membran wurde wie im Beispiel 15 hergestellt. Danach wurde die Membran mit 220 g einer Lösung von 5 g Phenylglycidylether in 190 g 60%-iger Ethanol-Lösung (60 Gewichtsprozent Ethanol, 40 Gewichtsprozent RO-Wasser) 1 h bei 40 °C behandelt. Die Membran wurde anschließend 2 mal 10 Minuten bei Raumtemperatur mit 5%iger Schwefelsäure-Lösung, 10 min mit 1 M NaCl-Lösung sowie 2 mal 5 min mit Ethanol behandelt und danach 10 Minuten mit fließendem Wasser gespült.

**Vergleichsbeispiel 3**

Überprüfung der kovalenten Bindung

Um zu überprüfen, ob die Polyamine kovalent an der Membran gebunden sind, wurden die Epoxy-Gruppen vor der Umsetzung mit Polyethylenimin hydrolysiert und dadurch deaktiviert. Die CA-Membran wurde wie im Beispiel 15 verseift und aktiviert. Danach wurde die Membran mit 5%iger Schwefelsäure-Lösung 3 h bei 50 °C behandelt und wie in Beispiel 16 mit Lupasol® WF umgesetzt.

Ladungsdichte Beispiel 16: 30,1 $\mu$eq/$cm^2$
Ladungsdichte Vergleichsbeispiel 3: 0,9 $\mu$eq/$cm^2$

**Vergleichsbeispiel 4**

Funktionalisierung der Membran mit Polyethylenimin über ein auf die Membran gepfropftes, epoxygruppenhaltiges Polymer

Eine Cellulosehydratmembran wurde gemäß EP 0 527 992 B1 (Beispiel 1, Beschichtungskonzentration 0,2 %, 5 % Glycidylmethacrylat, 0,1 % Natriumdithionit, Pfropfdauer 10 min) mit Glycidylmethacrylat gepfropft und nachfolgend mit Aceton und fließendem RO-Wasser gespült.

Diese Membran wurde mit einer 40%-igen wässrigen Lösung von Lupasol® FG (Polyethylenimin (PEI) der BASF AG, CAS-Nr. 25987-06-8, Molmasse 800 g/mol) unter Verwendung von 1 g Lösung pro Quadratzentimeter Membran 1 h bei 50 °C behandelt. Die Membran wurde anschließend 5 Minuten bei Raumtemperatur mit 5%iger Schwefelsäure-Lösung behandelt und danach 10 Minuten mit fließendem Wasser gespült.

Die Ergebnisse der Versuche mit Polyamin-Funktionalisierung sind in der nachstehenden Tabelle 3 gezeigt.

Tabelle 3

| Membran | Polymeres Amin | Mw | Durchfluss | Kapazität RSA* | Ladungsdichte | DNA Bindung |
|---|---|---|---|---|---|---|
| | | [g/mol] | [ml/(min*bar *cm$^2$)] | [mg/cm$^2$] | [$\mu$eq/cm$^2$] | [mg/cm$^2$] |
| Beispiel 15 | Lupasol® FG | 800 | 175 | 1,37 | 15,1 | 0,84 |
| Beispiel 16 | Lupasol® WF | 25.000 | 161 | 1,28 | 30,1 | 1,9 |
| Beispiel 17 | Lupasol® PN 50 | 1.000.000 | 85 | 1,98 | 12,6 | 1,12 |
| Beispiel 18 | Lupasol® WF | 25.000 | 168 | 0,98 | 16,7 | 0,68 |
| Beispiel 19 | Lupamin® 1595 | 10.000 | 224 | 0,80 | 9,7 | 0,51 |
| Beispiel 20 | Lupamin® 9095 | 430.000 | 229 | 1,93 | 4,0 | 0,45 |
| Beispiel 21 | Polyallylamin | 15.000 | 155 | 1,90 | 15,0 | 0,6 |
| Beispiel 22 | Polyallylamin | 150.000 | 191 | 1,40 | 12,0 | 0,56 |
| Beispiel 23 | Lupasol® WF | 25.000 | 167 | 0,99 | 13,2 | - |
| | | | | | | |
| Vergleich Sartobind® Q | - | - | 228 | 0,97 | 3,0 | 0,18 |
| Vergleichsbeispiel 3 | Lupasol® WF | 25.000 | 173 | - | 0,9 | - |
| Vergleichsbeispiel 4 | Lupasol® FG | 800 | 457 | 0,01 | 14,2 | 0,04 |
| * RSA: Rinderserumalbumin | | | | | | |

**Auswertung der Membranen (für die Beispiele 15 bis 23 und die Vergleichsbeispiele 3 und 4)**

Die Auswertung der erhaltenen Membranen erfolgte in der nachstehend beschriebenen Weise:

1) Durchflussbestimmung

Membranen mit einer aktiven Membranfläche von 12,5 cm$^2$ wurden jeweils in ein Gehäuse eingebaut und es wurde die Zeit für die Filtration von 100 ml 20 mM TRIS/HCl-Puffer bei pH 7,3 und bei 0,1 bar gemessen.

2) Bestimmung der statischen Bindungskapazität

Membranproben mit einer aktiven Membranfläche von jeweils 17,6 cm$^2$ wurden in 35 ml 20 mM TRIS/HCl-Puffer bei pH 7,3 3 x 5 min mit etwa 80 Umdrehungen pro Minute (Upm) geschüttelt. Danach wurden 35 ml einer 2-mg/ml-Rinderserumalbumin-(RSA)-Lösung (Bezugsquelle SERVA) in 20 mM TRIS/HCl-Puffer bei pH 7,3 12-18 Stunden bei 20-25 °C mit etwa 80 Upm geschüttelt. Anschließend wurden die Membranproben 2 x 15 Minuten mit jeweils 35 ml 20 mM TRIS/HCl-Puffer bei pH 7,3 gespült. Danach wurden die Membranproben in 20 ml 20 mM TRIS/HCl-Puffer (pH 7,3) + 1 M NaCl geschüttelt. Die Menge des eluierten Proteins wurde durch Messung der optischen Dichte (OD) bei 280 nm bestimmt.

3) Bestimmung der Ladungsdichte

3 Lagen Membran wurden in einen Membranhalter eingespannt. Der Membranstapel wies eine Membranfläche von 15 cm$^2$, eine Anströmfläche von 5 cm$^2$ und eine Betthöhe (Dicke des Membranstapels) von 750 $\mu$m in dem Membranhalter auf. Die Membranen in dem Membranhalter wurden mit 20 mM TRIS/HCl-Puffer bei pH =7,4 geflutet, um die Luft zu verdrängen und dann an eine FPLC-Anlage Äkta Explorer 100 der Firma General Electric Health Care angeschlossen. Danach wurden die Membranen bzw. der Membranstapel mit einem vier Schritte umfassenden Testprogramm hinsichtlich der Ladungsdichte untersucht. Die vier Schritte des Testprogramms sind nachfolgend angegeben:

1. Konditionieren der Membran mit 6 ml 1 M NaCl-Lösung in 20 mM TRIS / HCl bei pH =7,
2. Regenerieren der Membran mit 6 ml einer 1M-Lösung von NaOH in RO-Wasser,
3. Waschen der Membran mit 88 ml RO- Wasser und
4. Beladen der Membran mit 21 ml 10 mM HCl

Alle Schritte wurden bei einer Flußgeschwindigkeit von 10 ml/min durchgeführt. Bei allen Schritten wurde die Leitfähigkeit im Detektor hinter der Membraneinheit gemessen. Die Fläche oberhalb der so aufgezeichneten Durchbruchskurve wurde nach dem Abziehen des Totvolumens integriert und daraus die Ladungsdichte berechnet.

4) Bindung von DNS

3 Lagen Membran aus den Beispielen 15 bis 22 wurden in einen Membranhalter eingespannt. Der Membranstapel wies eine Membranfläche von 15 $cm^2$, eine Anströmfläche von 5 $cm^2$ und eine Betthöhe (Dicke des Membranstapels) von 750 $\mu$m in dem Membranhalter auf. Die Membranen in dem Membranhalter wurden mit 50 mM NaCl in 20 mM TRIS/HCl-Puffer (pH =7,4) geflutet, um die Luft zu verdrängen und dann an eine FPLC-Anlage Äkta Explorer 100 der Firma General Electric Health Care angeschlossen.

Danach wurden die Membranen bzw. der Membranstapel mit einem zwei Schritte umfassenden Testprogramm hinsichtlich der DNS-Bindung untersucht. Die zwei Schritte des Testprogramms sind nachfolgend angegeben:

1. Äquilibrieren der Membran mit 10 ml einer 50mM-NaCl-Lösung in 20 mM TRIS/HCl-Lösung (pH =7,4)
2. Beladen der Membran mit 100 $\mu$g/ml Lachssperma-DNS (Bezugsquelle VWR, Produktnummer 54653) in 50 mM NaCl in 20 mM TRIS/HCl-Lösung, bis die Konzentration im Detektor 10 % der Ausgangskonzentration betrug.

Alle Schritte wurden bei einer Flußgeschwindigkeit von 10 ml/min durchgeführt. Bei allen Schritten wurde die Absorption bei 260 nm im Detektor hinter der Membraneinheit gemessen. Die Fläche oberhalb der so aufgezeichneten Durchbruchskurve wurde nach dem Abziehen des Totvolumens integriert und daraus die DNS-Bindung bei 10 % Durchbruch berechnet.

5) Bindung von Wirtszellproteinen (HCP, "Host-Cell Proteins")

Für die Bestimmung der Bindung von Wirtszellproteinen wurde eine 10x konzentrierte HCP-Lösung in PBS-Puffer (pH =7,4) ohne Antikörper verwendet, hergestellt bei einem Kontrakthersteller in einem Mock-Lauf (Kultivierung einer Zelllinie ohne Antikörperproduktion) einer Ovarienzelllinie chinesischer Hamster. Die HCP-Lösung wurde 1:10 in 20 mM TRIS/HCl-Puffer (pH =7,4) verdünnt und die Leitfähigkeit wurde auf 10 mS/cm durch Zugabe von NaCl eingestellt. 1000 ml der verdünnten HCP-Lösung wurden zur Beladung der Membranen verwendet. Die HCP-Konzentration wurde mit Hilfe eines ELISA-Tests ("Enzyme-linked immunosorbent assay ELISA Cygnus CM015") nach den Herstellervorgaben bestimmt. Die Konzentration an Wirtszellproteinen (HCP) betrug 7 $\mu$g/ml.

3 Lagen Membran aus Beispiel 16 bzw. 21 wurden in einen Membranhalter eingespannt. Der Membranstapel wies eine Membranfläche von 15 $cm^2$, eine Anströmfläche von 5 $cm^2$ und eine Betthöhe (Dicke des Membranstapels) von 750 $\mu$m in dem Membranhalter auf. Die Membranen in dem Membranhalter wurden mit 20 mM TRIS/HCl-Puffer (pH =7,4) geflutet, um die Luft zu verdrängen und dann an eine FPLC-Anlage Äkta Explorer 100 der Firma General Electric Health Care angeschlossen.

Danach wurden die Membranen bzw. der Membranstapel mit einem vier Schritte umfassenden Testprogramm hinsichtlich der HCP-Bindung untersucht. Die vier Schritte des Testprogramms sind nachfolgend angegeben:

1. Äquilibrieren der Membran mit 10 ml 20 mM TRIS/HCl-Puffer (pH =7,4) mit einer Leitfähigkeit von 10 mS/cm,
2. Beladen der Membran mit 100 ml HCP-Lösung,
3. Waschen mit 10 ml 20 mM TRIS / HCl (pH =7,4, Leitfähigkeit 10 mS/cm) und
4. Eluieren mit 10 ml 1M NaCl in 20 mM TRIS/HCl-Puffer (pH =7,4).

Alle Schritte wurden bei einer Flußgeschwindigkeit von 10 ml/min durchgeführt. Bei allen Schritten wurde die Absorption bei 280 nm im Detektor hinter der Membraneinheit gemessen. Die Durchbruchskurven sind in der Figur 7 dargestellt.

6) Bindung von Modellviren

Der Bacteriophage ΦX174 mit einem Durchmesser von ca. 30 nm und einem isoelektrischen Punkt pI von 6,4-6,7 wurde als Modellvirus verwendet.

Phage: Bacteriophage ΦX174 (ATCC 13706-B1)

Wirtsorganismus: Escherichia coli C (ATCC 13706);

Begriffe/ Abkürzungen:

Pfu: Plaque Forming Units

LRV: Logarithmisches Rückhaltevermögen ("Logarithmic Reduction Value")
LF: Leitfähigkeit der Pufferlösung

Phagenlösung:

Der Phagenstamm mit $10^{10}$ Pfu/ml wird bei -70 °C gelagert. Die Phagen wurden im jeweiligen Puffer so verdünnt, dass ein Titer von $4x10^7$ pfu/ml vorlag. Vor der Beladung der Membranproben wurden die Ausgangslösungen über eine Sartopore® 2 Membran (0,1$\mu$m) filtriert.

Puffer A: 25mmol/l Tris/HCl pH=8,1, LF=1,38mS/cm
Puffer B: 25mmol/l Tris/HCl + 50mmol/l Natriumchlorid pH=8,1, LF=6,7mS/cm
Puffer C: 25mmol/l Tris/HCl + 150mmol/l Natriumchlorid pH=8,1, LF=16,8mS/cm

Membranen:

Vergleichsbeispiel: Sartobind® Q Membran
Beispiel 21: Polyallylamin
Beispiel 16: Polyethylenimin

Von den Membranproben wurden 30-mm-Scheiben gestanzt und 3-lagig in einen Membranhalter (LP15) eingespannt. Der Membranstapel wies eine Membranfläche von 15 $cm^2$, eine Anströmfläche von 5 $cm^2$ und eine Betthöhe (Dicke des Membranstapels) von 750 $\mu$m in dem Membranhalter auf.
Berechnungen:

$$LRV = \log_{10}\left(\frac{C_{Ausganslösung}}{C_{Durchlauf}}\right)$$

$c_{Ausgangslösung}$ = Phagentiter in der Ausgangslösung [pfu/ml]
$c_{Durchlauf}$ = Phagentiter im Durchlauf [pfu/ml]

Durchführung:

Die Versuche wurden an einer Mehrkanalpumpe durchgeführt, sodass eine Membranprobe in drei LP15 eingebaut und parallel mit Phagen im Puffersystem A (ohne Salz), B (mit 50 mmol/l NaCl) und C (150 mmol/l NaCl) beaufschlagt wurde. Es wurde ein Volumen von 800 ml Phagenlösung bei einem Fluss von 20 ml/min, filtriert. Der Durchlauf wurde in 200-ml-Fraktionen aufgefangen. Jeder Versuch wurde doppelt durchgeführt. Die Ausgangslösungen und die Durchläufe wurden unter der Clean-Bench verdünnt und mit dem Wirtsorganismus Escherichia coli C zusammengegeben. Nach der Inkubationszeit von 10 min wurden die Proben auf Agar ausplattiert und über Nacht bei 37 °C bebrütet. Am nächsten Tag wurden die Plaques ausgezählt und der Titer berechnet.
Die Ergebnisse der Versuche sind in der nachstehenden Tabelle 4 gezeigt.

Tabelle 4: LRV nach 800 ml Phagenlösung mit einem Titer von $4x10^7$ pfu/ml

| Membrane | 0 mmol/l NaCl | 50 mmol/l NaCl | 150 mmol/l NaCl |
|---|---|---|---|
| Vergleichsbeispiel Sartobind® Q | 2,5 | 0 | 0 |
| Beispiel 16 | 4,5 | 4 | 3,5 |
| Beispiel 21 | 5 | 5 | 4,5 |

## Patentansprüche

1. Verfahren zur Herstellung einer vernetzten Cellulosehydrat-Membran mit einer porösen Doppelstruktur, welche besteht aus

- Mikroporen mit einem Durchmesser im Bereich von >100 nm bis 20 μm, und
- Ultraporen mit einem Durchmesser von < 100 nm, die für Dextranblau mit einem mittleren Molekulargewicht Mw von 2.000.000 nicht zugänglich sind,

wobei der Anteil des Volumens der Ultraporen an dem für Wasser zugänglichen Gesamtporenvolumen mehr als 15% beträgt, umfassend die Schritte:

- Bereitstellen einer Celluloseester-Membran mit einem Porendurchmesser von 0,1 bis 20 μm;
- gegebenenfalls Vorbehandeln der Celluloseester-Membran in einem Medium bei einer Temperatur von 20°C bis 100°C;
- Verseifen der gegebenenfalls vorbehandelten Celluloseester-Membran unter quellenden Bedingungen in einem wässrigen Medium, wobei das wässrige Medium Natrium- oder Lithiumhydroxid enthält, und
- nachfolgendes Vernetzen der verseiften Membran mit mindestens einem Vernetzungsmittel, welches bzw. welche mindestens 2 funktionelle Gruppen im Molekül aufweist bzw. aufweisen, die mit den Hydroxylgruppen der Cellulose reaktiv sind,

wobei die Konzentration des Natrium- oder Lithiumhydroxids im Verseifungsmedium 0,4 Gew.% bis 50 Gew.%, bezogen auf das Verseifungsmedium, beträgt.

**2.** Verfahren nach Anspruch 1, wobei das Verseifungsmedium mindestens ein quellungsbeeinflussendes Additiv enthält.

**3.** Verfahren nach Anspruch 1 oder 2, wobei funktionelle Gruppen an die Membran gebunden werden.

**4.** Verfahren nach Anspruch 3, wobei die funktionellen Gruppen während der Vernetzung oder nach der Vernetzung eingeführt werden.

**5.** Vernetzte Cellulosehydrat-Membran mit einer porösen Doppelstruktur, hergestellt nach dem Verfahren des Anspruchs 1, welche besteht aus

- Mikroporen mit einem Durchmesser im Bereich von >100 nm bis 20 μm, und
- Ultraporen mit einem Durchmesser von < 100 nm, die für Dextranblau mit einem mittleren Molekulargewicht Mw von 2.000.000 nicht zugänglich sind,

wobei der Anteil des Volumens der Ultraporen an dem für Wasser zugänglichen Gesamtporenvolumen mehr als 15% beträgt.

**6.** Vernetzte Cellulosehydrat-Membran nach Anspruch 5, wobei mindestens eine funktionelle Gruppe an die Membran gebunden ist.

**7.** Vernetzte Cellulosehydrat-Membran nach Anspruch 6, wobei die mindestens eine funktionelle Gruppe ein Ligand ist bzw. Liganden sind, der bzw. die befähigt ist bzw. sind, mit in Fluiden enthaltenen Adsorbenden Wechselwirkungen einzugehen.

**8.** Vernetzte Cellulosehydrat-Membran nach Anspruch 7, wobei die Liganden anionische und kationische Gruppen umfassen.

**9.** Vernetzte Cellulosehydrat-Membran nach Anspruch 8, wobei die kationischen Gruppen ausgewählt sind aus der Gruppe der primären, sekundären, tertiären und/oder quaternären Amine.

**10.** Vernetzte Cellulosehydrat-Membran nach Anspruch 9, wobei die primären, sekundären und/oder tertiären Amine polymere Verbindungen mit linearen und/oder verzweigten und/oder cyclischen Strukturen sind.

**11.** Vernetzte Cellulosehydrat-Membran nach Anspruch 10, wobei die polymeren Verbindungen ausgewählt sind aus der Gruppe der Polyalkylenimine mit einer Molmasse zwischen 800 und 1.000.000 g/mol.

**12.** Vernetzte Cellulosehydrat-Membran nach Anspruch 10, wobei die polymeren Verbindungen ausgewählt sind aus der Gruppe der Polyallylamine mit einer Molmasse zwischen 3.000 und 150.000 g/mol.

**13.** Vernetzte Cellulosehydrat-Membran nach Anspruch 10, wobei die polymeren Verbindungen ausgewählt sind aus der Gruppe der Polyvinylamine mit einer Molmasse zwischen 5.000 und 500.000 g/mol.

**14.** Vernetzte Cellulosehydrat-Membran nach Anspruch 7, wobei mindestens zwei strukturell unterschiedliche Liganden an die Membran gebunden sind.

**15.** Vernetzte Cellulosehydrat-Membran nach einem der Ansprüche 6 bis 14, wobei die mindestens eine funktionelle Gruppe ein Teil eines Abstandshalter ist, der an die Cellulosehydrat-Membran gebunden ist.

**16.** Verwendung der Membran nach einem der Ansprüche 7 bis 15 als Adsorptionsmembran.

**17.** Vorrichtung zur Membranchromatographie, umfassend mindestens eine vernetzte Cellulosehydrat-Membran nach einem oder mehreren der Ansprüche 7 bis 15.

## Claims

**1.** A method for producing a crosslinked cellulose hydrate membrane having a porous double structure, consisting of

- micropores having a diameter in the range from >100 nm to 20 $\mu$m, and
- ultrapores having a diameter of <100 nm, which are not accessible to Blue Dextran having an average molecular weight Mw of 2 000 000,

wherein the fraction of the volume of the ultrapores is more than 15% of the entire pore volume accessible to water, comprising the steps of:

- providing a cellulose ester membrane having a pore diameter of from 0.1 to 20 $\mu$m;
- optionally pretreating the cellulose ester membrane in a medium at a temperature of from 20°C to 100°C;
- hydrolyzing the optionally pretreated cellulose ester membrane under swelling conditions in an aqueous medium containing sodium hydroxide or lithium hydroxide;
- subsequently, crosslinking the hydrolyzed membrane with at least one crosslinking agent which has at least 2 functional groups in the molecule which are reactive with the hydroxyl groups of the cellulose,

wherein the concentration of the sodium hydroxide or lithium hydroxide in the hydrolysis medium is from 0.4% by weight to 50% by weight, based on the hydrolysis medium.

**2.** The method according to claim 1, wherein the hydrolysis medium comprises at least one swelling-influencing additive.

**3.** The method according to claim 1 or 2, wherein functional groups become bonded to the membrane.

**4.** The method according to claim 3, wherein the functional groups are introduced during the crosslinking or after the crosslinking.

**5.** A crosslinked cellulose hydrate membrane having a porous double structure produced by the method according to claim 1, consisting of

- micropores having a diameter in the range from >100 nm to 20 $\mu$m, and
- ultrapores having a diameter in the range of <100 nm, which are not accessible to Blue Dextran having an average molecular weight Mw of 2 000 000,

wherein the fraction of the volume of the ultrapores is more than 15% of the entire pore volume accessible to water.

**6.** The crosslinked cellulose hydrate membrane according to claim 5, wherein at least one functional group is bonded to the membrane.

**7.** The crosslinked cellulose hydrate membrane according to claim 6, wherein the at least one functional group is/are ligand(s) which is/are capable of entering into interactions with adsorbates present in fluids.

8. The crosslinked cellulose hydrate membrane according to claim 7, wherein the ligands comprise anionic and cationic groups.

9. The crosslinked cellulose hydrate membrane according to claim 8, wherein the cationic groups are selected from the group of the primary, secondary, tertiary, and/or quaternary amines.

10. The crosslinked cellulose hydrate membrane according to claim 9, wherein the primary, secondary, and/or tertiary amines are polymeric compounds having linear and/or branched and/or cyclic structures.

11. The crosslinked cellulose hydrate membrane according to claim 10, wherein the polymeric compounds are selected from the group of the polyalkyleneimines having a molar mass between 800 and 1 000 000 g/mol.

12. The crosslinked cellulose hydrate membrane according to claim 10, wherein the polymeric compounds are selected from the group of the polyallylamines having a molar mass between 3000 and 150 000 g/mol.

13. The crosslinked cellulose hydrate membrane according to claim 10, wherein the polymeric compounds are selected from the group of the polyvinylamines having a molar mass between 5000 and 500 000 g/mol.

14. The crosslinked cellulose hydrate membrane according to claim 7, wherein at least two structurally different ligands are bonded to the membrane.

15. The crosslinked cellulose hydrate membrane according to any one of claims 6 to 14, wherein the at least one functional group is part of a spacer which is bonded to the cellulose hydrate membrane.

16. Use of the membrane according to any one of claims 7 to 15 as an adsorption membrane.

17. An apparatus for membrane chromatography, comprising at least one crosslinked cellulose hydrate membrane according to any one of claims 7 to 15.

**Revendications**

1. Procédé pour la fabrication d'une membrane en hydrate de cellulose réticulé présentant une structure double poreuse, ladite membrane étant constituée par

   - des micropores ayant un diamètre dans la gamme allant de >100 nm à 20 μm, et
   - des ultra-pores ayant un diamètre de <100 nm qui ne sont pas accessibles au bleu de dextrane ayant un poids moléculaire moyen de 2000000, la fraction volumétrique des ultra-pores par rapport au volume total de pores accessibles à l'eau étant de plus de 15%,

   procédé comportant les étapes :

   - mise à disposition d'une membrane d'ester de cellulose ayant un diamètre de pore de 0,1 à 20 μm ;
   - éventuellement prétraitement de la membrane d'ester de cellulose dans un milieu à une température de 20°C à 100°C ;
   - saponification de la membrane d'ester de cellulose éventuellement prétraitée dans des conditions de gonflement en milieu aqueux comportant de l'hydroxyde de sodium ou de lithium, et
   - ensuite réticulation de la membrane saponifiée à l'aide d'au moins un agent de réticulation qui comporte dans la molécule au moins deux groupes fonctionnels qui sont réactifs par rapport aux groupes hydroxyles de la cellulose,
   - la concentration en hydroxyde de sodium ou de lithium dans le milieu de saponification étant de 0,4 % en poids à 50 % en poids, par rapport au milieu de saponification.

2. Procédé selon la revendication 1 dans lequel le milieu de saponification contient au moins un additif influençant le gonflement.

3. Procédé selon la revendication 1 ou 2 dans lequel des groupements fonctionnels sont liés à la membrane.

**4.** Procédé selon la revendication 3 dans lequel les groupements fonctionnels sont introduits pendant ou après la réticulation.

**5.** Membrane d'hydrate de cellulose réticulé présentant une structure double poreuse, obtenue selon le procédé de la revendication 1, ladite membrane étant constituée

- de micropores ayant un diamètre dans la gamme allant de >100 nm à 20 μm, et
- d'ultra-pores ayant un diamètre de <100 nm qui ne sont pas accessibles au bleu de dextrane ayant un poids moléculaire moyen de 2000000, la fraction volumétrique des ultra-pores par rapport au volume total de pores accessibles à l'eau étant de plus de 15%.

**6.** Membrane d'hydrate de cellulose réticulé selon la revendication 5 dans laquelle au moins un groupement fonctionnel est lié à la membrane.

**7.** Membrane d'hydrate de cellulose réticulé selon la revendication 6 dans laquelle ledit au moins un groupement fonctionnel est un ligand apte à interagir avec des adsorbants contenus dans des fluides.

**8.** Membrane d'hydrate de cellulose réticulé selon la revendication 7 dans laquelle les ligands comportent des groupements anioniques et cationiques.

**9.** Membrane d'hydrate de cellulose réticulé selon la revendication 8 dans laquelle les groupements cationiques sont choisis dans le groupe des amines primaires, secondaires, tertiaires et/ou quaternaires.

**10.** Membrane d'hydrate de cellulose réticulé selon la revendication 9 dans laquelle les amines primaires, secondaires et/ou tertiaires sont des composés polymères de structure linéaire et/ou ramifiée et/ou cyclique.

**11.** Membrane d'hydrate de cellulose réticulé selon la revendication 10 dans laquelle les composés polymères sont choisis dans le groupe des polyalkyleneamines ayant une masse molaire comprise entre 800 et 1000000 g/mol.

**12.** Membrane d'hydrate de cellulose réticulé selon la revendication 10 dans laquelle les composés polymères sont choisis dans le groupe des polyallylamines ayant une masse molaire comprise entre 3000 et 150000 g/mol.

**13.** Membrane d'hydrate de cellulose réticulé selon la revendication 10 dans laquelle les composés polymères sont choisis dans le groupe des polyvinylamines ayant une masse molaire comprise entre 5000 et 500000 g/mol.

**14.** Membrane d'hydrate de cellulose réticulé selon la revendication 7 dans laquelle au moins deux ligands structurellement différents sont liés à la membrane.

**15.** Membrane d'hydrate de cellulose réticulé selon l'une des revendications 6 à 14 dans laquelle ledit au moins un groupement fonctionnel est une partie d'un groupement d'écartement qui est lié à la membrane d'hydrate de cellulose.

**16.** Membrane d'hydrate de cellulose réticulé selon l'une des revendications 7 à 15 servant de membrane d'adsorption.

**17.** Dispositif pour le chromatographie sur membrane comportant au moins une membrane d'hydrate de cellulose réticulé selon l'une ou plusieurs des revendications 7 à 15.

Figur 1

a)
b)
c)
Träger
Adsorbend
Polymerbeschichtung
Ultraporen

Figur 2

10 µm
Durchlaufende Mikroporen
grobe Struktur aus relativ dicken Fasern der Cellulose bzw. deren Agglomeraten, adsorptiv unwirksam
feiner verteiltem, faser- oder clusterartigen Membranmaterial
An der adsorptiv wirksamen Polymerschicht gebundenes Lysozym

Figur 3

10 µm
Durchlaufende Mikroporen
grobe Struktur aus relativ dicken Fasern der Cellulose bzw. deren Agglomeraten, mit gebundenem Lysozym
feiner verteiltem, faser- oder clusterartigen Membranmaterial mit gebundenem Lysozym

Figur 4

10 µm

Durchlaufende Mikroporen

grobe Struktur aus relativ dicken Fasern der Cellulose bzw. deren Agglomeraten mit für Biomoleküle zugänglichen Ultraporen mit gebundenem Lysozym

feiner verteiltem, faser- oder clusterartigen Membranmaterial mit für Biomoleküle zugänglichen Ultraporen mit gebundenem Lysozym

Figur 5

50%
40%
30%
20%
10%
0%
Vp [%]
A B C D E F 1 2 3 4 5 6
Membran

Figur 6

HO—S—OH
O
O
H₂N
NH
NH
HO
NH₂
CH₂
H₂C
NH₂
NH₂
NH₂
HO

Figur 7

30
25
20
15
10
5
0
OD 280 nm [mAu]
Sartobind Q
Beispiel 16
Beispiel 21
0
20
40
60
80
100
HCP Beladung [ml]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2007017085 A2
- EP 0586268 B1
- WO 2008008872 A2
- US 7396465 B2
- US 20070256970 A1
- EP 1224975 B1
- EP 0343387 B1
- WO 03015902 A2
- US 6103121 A
- DE 4323913 A1
- WO 2008095709 A1
- US 5739316 A
- EP 0527992 B1


### In der Beschreibung aufgeführte Nicht-Patentliteratur


- **GREG T. HERMANSON ; A. KRISHNA MALLIA ; PAUL K. SMITH.** Immobilized Affinity Ligand Techniques. Academic Press, INC, 1992
- **U.-J. KIM ; S. KUGA.** *Journal of Chromatography A,* 2002, vol. 955, 191-196
- **GREG T. ; HERMANSON, A. ; KRISHNA MALLIA ; PAUL K. SMITH.** Immobilized Affinity Ligand Techniques. Academic Press, INC, 1992